# EUROPEAN PATENT APPLICATION

(11) **EP 2 878 668 A2**
(43) Date of publication of application: **03.06.2015**
(21) Application number: 14195391.9
(22) Date of filing: 28.11.2014
(51) Int. Cl.: C12N 11/04, B01L 3/00, C12Q 1/68

(54) **Container for nucleic acid amplification reaction, cartridge for nucleic acid amplification reaction, and cartridge kit for nucleic acid amplification reaction**

(30) Priority: 29.11.2013 JP 2013248576
(71) Applicant: Seiko Epson Corporation, Shinjuku-ku Tokyo (JP)
(72) Inventor: Idegami, Kotaro, Nagano, 392-8502 (JP); Murayama, Toshiro, Nagano, 392-8502 (JP)
(74) Representative: Hoffmann Eitle

(57) **Abstract**

A container for nucleic acid amplification reaction includes a freeze-dried nucleic acid amplification reaction reagent, an oil, and a container that contains the freeze-dried nucleic acid amplification reaction reagent and the oil, in which the freeze-dried nucleic acid amplification reaction reagent is held in the oil, and a cartridge for nucleic acid amplification reaction and a cartridge kit for nucleic acid amplification reaction are configured using the same.

## Description

### BACKGROUND

### 1. Technical Field

The present invention relates to a container for nucleic acid amplification reaction, a cartridge for nucleic acid amplification reaction, and a cartridge kit for nucleic acid amplification reaction.

### 2. Related Art

In recent years, as a result of development of technologies utilizing genes, medical treatments utilizing genes such as genetic diagnosis or gene therapy are drawing attention. In addition, many methods utilizing genes in determination of breed varieties or breed improvement have also been developed in the fields of agriculture and stock-breeding. As technologies for utilizing genes, technologies such as a polymerase chain reaction (PCR) method are widely used. Nowadays, PCR becomes an indispensable technology in elucidation of information on biological materials. PCR is a method in which thermal cycling is applied to a solution (reaction solution) that includes a nucleic acid as an amplification target (target nucleic acid) and a reagent to thereby amplify the target nucleic acid. As the thermal cycling of PCR, a method of applying thermal cycling at temperatures of two stages or three stages is usually adopted.

On the other hand, for the diagnosis of infectious diseases such as influenza at the site of medical care, the use of simple test kits of immunochromatography and the like is the mainstream under the present circumstances. However, in such rapid tests, the accuracy is sometimes insufficient, so that it is desired to apply PCR, from which higher test accuracy can be expected, to the diagnosis of infectious diseases. Moreover, in a general outpatient practice or the like in medical institutions, since the examination time is limited, the time that can be spent for a test is limited to a short time. Therefore, the reality is that a test for influenza, for example, is performed by a simple test such as immunochromatography, at the expense of test accuracy, for shortening the time.

Because of such circumstances, for realizing a test by PCR, from which higher accuracy can be expected, at the site of medical care, the time required for the reaction needs to be shortened. As an apparatus for performing PCR reaction in a short time, for example, JP-A-2009-136250, discloses a biological sample reaction apparatus that rotates a biological sample reaction chip filled with a reaction solution and a liquid that is not miscible with the reaction solution and has a specific gravity smaller than that of the reaction solution, about an axis of rotation in a horizontal direction to thereby move the reaction solution for applying thermal cycling. In addition, as methods of PCR, a method of using magnetic beads (JP-A-2009-207459), a method of performing thermal cycling of PCR by moving a droplet in a temperature change region on a substrate using magnetic beads as moving means of the droplet (JP-A-2008-012490), and the like are disclosed.

### SUMMARY

An advantage of some aspects of the invention is to provide a container for nucleic acid amplification reaction, a cartridge for nucleic acid amplification reaction, and a cartridge kit for nucleic acid amplification reaction.

An aspect of the invention is directed to a container for nucleic acid amplification reaction including a freeze-dried nucleic acid amplification reaction reagent, an oil, and a container that contains the nucleic acid amplification reaction reagent and the oil, in which the nucleic acid amplification reaction reagent is held in the oil. The oil may be subjected to dehydration treatment. It is preferable that the freeze-dried nucleic acid amplification reaction reagent be cake-like. It is preferable that the freeze-dried nucleic acid amplification reaction reagent be a porous substance having a hole having a diameter of 20 µm or less. The nucleic acid amplification reaction reagent may contain a DNA polymerase and dNTP, a primer for nucleic acid amplification reaction, or a reverse transcriptase. Further, the nucleic acid amplification reaction reagent and the oil for nucleic acid amplification reaction may be separated by a solid wax.

Another aspect of the invention is directed to a cartridge for nucleic acid amplification reaction including a tube that is internally provided with, in the following order, in a longitudinal direction, a first plug composed of an oil, a second plug composed of a first washing liquid, which is phase-separated from an oil and is used for washing a fine particle having nucleic acids bound thereto, third plug composed of an oil, a fourth plug composed of an eluent which is phase-separated from an oil and is used for eluting the nucleic acids from a fine particle having the nucleic acids bound thereto, and a fifth plug composed of an oil, and a container for nucleic acid amplification reaction that is disposed to communicate with an end portion of the tube on a side on which the fifth plug is disposed and contains an oil, in which a freeze-dried nucleic acid amplification reaction reagent does not dissolve and is held in the oil in the container for nucleic acid amplification reaction. It is preferable that the nucleic acid amplification reaction reagent be disposed on an end portion of the container for nucleic acid amplification reaction opposite to the side communicating with the tube. It is preferable that the end portion of the container for nucleic acid amplification reaction opposite to the side communicating with the tube have a tapered shape. It is preferable that the specific gravity of the oil in the container for nucleic acid amplification reaction be smaller than the specific gravity of the eluent and the viscosity of the oil in the container for nucleic acid amplification reaction be 50 cs or less. The cartridge for nucleic acid amplification reaction may further include a pushing unit which is mounted on an opening portion of the tube on a side on which the first plug is disposed to push out the eluent from the end portion of the tube on the side on which the fifth plug is disposed to the container for nucleic acid amplification reaction. A tip end of the tube on the side on which the fifth plug is disposed may be brought into contact with the oil in the container for nucleic acid amplification reaction. The oil in the container for nucleic acid amplification reaction may be subjected to dehydration treatment. The nucleic acid amplification reaction reagent may contain a DNA polymerase and dNTP. The nucleic acid amplification reaction reagent may contain a primer for nucleic acid amplification reaction and the eluent may contain a primer for nucleic acid amplification reaction. The nucleic acid amplification reaction reagent may contain a reverse transcriptase. The nucleic acid amplification reaction reagent and the oil may be separated by a solid wax. The amount of the nucleic acid amplification reaction reagent for freeze-drying the nucleic acid amplification reaction reagent may be smaller than the amount of the eluent.

Still another aspect of the invention is directed to a cartridge kit for nucleic acid amplification reaction including the cartridge for nucleic acid amplification reaction according to any one of the above aspects, and a tank for introducing a nucleic acid-binding solid-phase carrier into the tube. The tank may contain a solution for extracting nucleic acids and the nucleic acid-binding solid-phase carrier. The tank may have an opening portion and a removable cap on the opening portion. The opening portion of the tank may be configured to be mountable on the opening portion of the tube on the side on which the first plug is disposed.

### BRIEF DESCRIPTION OF THE DRAWINGS

The invention will be described with reference to the accompanying drawings, wherein like numbers reference like elements.
Figs. 1A and 1B are views illustrating a cartridge.
Figs. 2A to 2C are views illustrating the operation of the cartridge.
Figs. 3A to 3D are views illustrating a tank.
Fig. 4 is a view illustrating a fixing claw, a guide plate, and a mounting portion.
Figs. 5A and 5B are views illustrating the vicinity of a PCR container.
Fig. 6A is a perspective view illustrating the inner configuration of a PCR apparatus and Fig. 6B is a side view illustrating the main configuration of the PCR apparatus.
Fig. 7 is a block diagram illustrating the PCR apparatus.
Fig. 8A is a view illustrating a rotary body and Fig. 8B is a view illustrating a state in which the cartridge is mounted on the mounting portion of the rotary body.
Figs. 9A to 9D are views illustrating states of the PCR apparatus when the cartridge is mounted.
Fig. 10 is a conceptual view of behavior of magnetic beads when magnets are moved downward.
Figs. 11A to 11C are views illustrating nucleic acid elution treatment.
Fig. 12 is a conceptual view of behavior of magnetic beads when the magnets are oscillated.
Fig. 13 is a table showing whether or not the magnets are oscillated.
Figs. 14A to 14C are views illustrating droplet forming treatment.
Figs. 15A to 15D are views illustrating thermal cycling treatment.
Fig. 16 is a view illustrating a kit for nucleic acid extraction and a device obtained by combining each component of the kit used in an example.
Fig. 17 is a graph showing the results of real-time PCR in an example.
Figs. 18A and 18B are graphs showing a comparison result of storage stability of a nucleic acid amplification solution in an example.
Figs. 19A to 19D are schematic views when a freeze-dried nucleic acid amplification reaction reagent is fixed to a tube in an embodiment.
Figs. 20A to 20C are schematic views illustrating a method for dissolving the freeze-dried nucleic acid amplification reaction reagent by using the configuration illustrated in Fig. 19A.
Fig. 21 is a graph showing the results of RT-PCR using a reagent obtained by dissolving a nucleic acid amplification reagent into an eluent using the method illustrated in Figs. 20A to 20C in an example.

### DESCRIPTION OF EXEMPLARY EMBODIMENTS

Hereinafter, embodiments of the invention will be described. The objects, features, advantages, and ideas of the invention are apparent to those skilled in the art from the description of this specification, and those skilled in the art can easily reproduce the invention from the description of this specification. The embodiments of the invention described below, which are given for the purpose of illustration or description, and the invention is not limited thereto. It is obvious to those skilled in the art that various modifications and changes may be made based on the description of the specification without departing from the scope of the invention as defined by the appended claims.

### First Embodiment

According to a first embodiment of the invention, there is provided a container for nucleic acid amplification reaction including a freeze-dried nucleic acid amplification reaction reagent, an oil, and a container for accommodating the nucleic acid amplification reaction reagent and the oil, in which the nucleic acid amplification reaction reagent is held in the oil. It is preferable that the freeze-dried nucleic acid amplification reaction reagent not dissolve in the oil.

The container that is included in the container for nucleic acid amplification reaction is not particularly limited and for example, a small tube of 0.2 mL to 1.5 mL that can be directly used as a nucleic acid amplifier of a PCR apparatus or the like is preferably used.

In the container, the freeze-dried nucleic acid amplification reaction reagent is held in the oil. The nucleic acid amplification reaction reagent contains at least a DNA polymerase and dNTP. However, it is preferable that the reagent contain a primer for nucleic acid amplification reaction and/or a probe for nucleic acid amplification reaction. In addition, the freeze-dried nucleic acid amplification reaction reagent may contain a reverse transcriptase. In this case, the primer for reverse transcription may be contained separately from the primer for nucleic acid amplification reaction or may be used together with the primer for nucleic acid amplification reaction.

The DNA polymerase is not particularly limited and is preferably a heat-resistant enzyme or an enzyme for use in PCR, and there are a great number of commercially available products such as Taq polymerase, Tfi polymerase, Tth polymerase, and a modified form thereof. However, the DNA polymerase by which hot start can be performed is preferably used. Further, the reverse transcriptase is also not particularly limited and for example, a reverse transcriptase derived from avian myeloblast virus, Ras associated virus type 2, mouse molony murine leukemia virus, or human immunodefficiency virus type 1, or the like can be used. However, a heat-resistant enzyme is preferably used.

The concentrations of the dNTP and the salt may be set to concentrations suitable for an enzyme to be used, however, the concentration of the dNTP may generally be set to 10 µM to 1000 µM and preferably 100 µM to 500 µM, the concentration of Mg²⁺ may be set to 1 mM to 100 mM and preferably 5 mM to 10 mM, and the concentration of Cl⁻ may be set to 1 mM to 2000 mM and preferably 200 mM to 700 mM. The total ion concentration is not particularly limited, and may be higher than 50 mM, and is preferably higher than 100 mM, more preferably higher than 120 mM, still more preferably higher than 150 mM, and further still more preferably higher than 200 mM. The upper limit thereof is preferably 500 mM or less, more preferably 300 mM or less, and still more preferably 200 mM or less. Each oligonucleotide as the primer is used in an amount of 0.1 µM to 10 µM, and preferably 0.1 µM to 1 µM.

The freeze-drying of the nucleic acid amplification reaction reagent may be performed by a typical method and for example, the nucleic acid amplification reaction reagent is added to a buffer for nucleic acid amplification reaction in an amount to cause a reaction in the container used as the container for nucleic acid amplification reaction, quickly frozen, pressure-reduced, and left for a predetermined period of time. The amount of the buffer for nucleic acid amplification reaction is not particularly limited and may be 5 µL, preferably 2.5 µL, and more preferably 1.6 µL. As the amount of buffer becomes smaller, the nucleic acid amplification reaction reagent is firmly fixed to the bottom of the container in a small size. The temperature when the reagent is quickly frozen is not particularly limited and a temperature in a range of -10°C to -160°C is preferable and a temperature of about -80°C is most preferable. The freeze-dried nucleic acid amplification reaction reagent is spongy or cake-like, and is preferably a porous substance having a large number of micro pores (air holes) in which bubbles are gathered therein. Thus, good solubility is achieved. As the quicker the reagent is frozen, the finer the micro pores are, and thus, the storage property is further enhanced. The average void diameter of the pores is (for example, the diameters of the pores of the cross section are measured a predetermined number of times under a microscope to obtain the average value thereof) preferably 20 µm or less. The pressure at the time of pressure reduction is not particularly limited and is preferably 100 mmHg or less, and most preferably 20 mmHg or less. The period in which the low pressure is maintained is also not particularly limited and is preferably 2 hours to 24 hours, and most preferably about 8 hours. The amount of the nucleic acid amplification reaction reagent solution for the freeze-drying the nucleic acid amplification reaction reagent is preferably smaller than the amount of an aqueous solution for dissolving the nucleic acid amplification reaction reagent.

In this manner, the nucleic acid amplification reaction reagent is fixed to the bottom of the container by freeze-drying the nucleic acid amplification reaction reagent in the container used as the container for nucleic acid amplification reaction. Then, an oil is added. It is preferable to add the oil so as to fill the container. The freeze-dried nucleic acid amplification reaction reagent is weak in moisture and cannot be stored stably for a long period of time in a state of moisture. Thus, it is preferable to dehydrate the oil with silica gel or the like in advance and the oil is preferably added in a globe box or the like. Further, it is preferable that the container be centrifuged gently to remove bubbles after the oil is added.

Before the oil is added to the freeze-dried nucleic acid amplification reaction reagent, a dissolved wax is added, the wax is solidified, and then the oil is added. Then, the freeze-dried nucleic acid amplification reaction reagent can be prevented from diffusing in the oil. Here, the wax is an organic material that is a solid at room temperature and is a liquid when heated. However, the wax to be used in the invention has a melting point of 31°C or higher, preferably 36°C or higher, more preferably 41°C or higher, and still more preferably 46°C or higher, and 100°C or lower, preferably 90°C or lower, more preferably 80°C or lower, and still more preferably 70°C or lower and preferably includes neutral fat, higher fatty acid, hydrocarbon, or the like. The wax is not particularly limited and examples thereof include petroleum-derived waxes such as paraffin wax and microcrystalline wax, animal waxes such as beeswax, lanolin, and spermaceti, and vegetable waxes such as carnauba wax, rosin, candelilla wax, and Japan wax. In addition, El Crysta (registered trademark, manufactured by Idemitsu Kosan Co., Ltd.), Nissan Electrabel Thor (registered trademark, manufactured by NOF CORPORATION), Poem (registered trademark, manufactured by Riken Vitamin Co., Ltd.), Rikemal (registered trademark, manufactured by Riken Vitamin Co., Ltd.), Neowax (registered trademark, manufactured by YASUHARA CHEMICAL CO.,LTD.), HI-WAX (registered trademark, manufactured by Mitsui Chemicals, Inc.), Silicon Wax (registered trademark, manufactured by Toray Dow Corning Silicone Co.), or the like can be used.

When the container included in the container for nucleic acid amplification reaction can be used as a nucleic acid amplifier of a PCR apparatus or the like, the container for nucleic acid amplification reaction containing the nucleic acid amplification reaction reagent and the oil can be used for nucleic acid amplification reaction as it is. Specifically, an appropriate amount of pure water or a buffer including the DNA to be amplified may be added so that the freeze-dried nucleic acid amplification reaction reagent is immersed therein to start nucleic acid amplification reaction. The amount of pure water or a buffer can be easily determined such that the final concentration of salts or the like is suitable for nucleic acid amplification reaction. Before the reaction, heat treatment or shaking treatment may be performed to dissolve the nucleic acid amplification reaction reagent sufficiently.

When the freeze-dried nucleic acid amplification reaction reagent includes a reverse transcriptase, an appropriate amount of pure water or a buffer including RNA may be added so that the freeze-dried nucleic acid amplification reaction reagent is immersed therein. In this case, reverse transcription reaction can be performed and then nucleic acid amplification reaction can be performed.

When the freeze-dried nucleic acid amplification reaction reagent is covered with a solid wax in the container for nucleic acid amplification reaction, the wax is heated to the temperature at which the wax dissolves to dissolve the wax and then an appropriate amount of pure water or a buffer including the nucleic acids may be added as described above.

### Second Embodiment

First, a cartridge that is mounted in a PCR apparatus 50 (nucleic acid amplification reaction apparatus) will be described and then the configuration and operation of the PCR apparatus 50 according to an embodiment will described.

### Cartridge 1

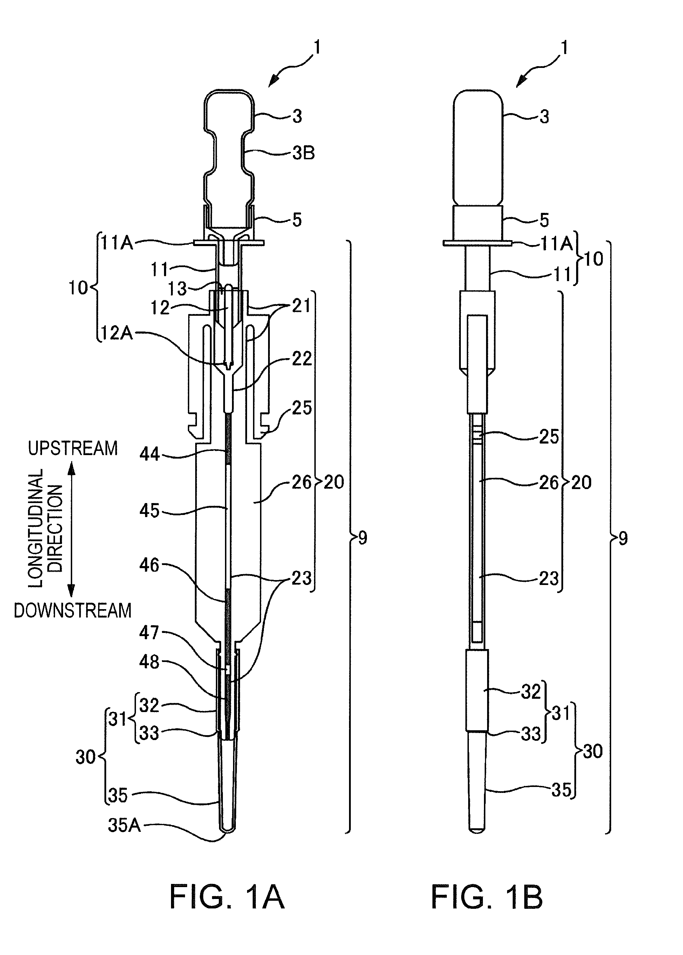

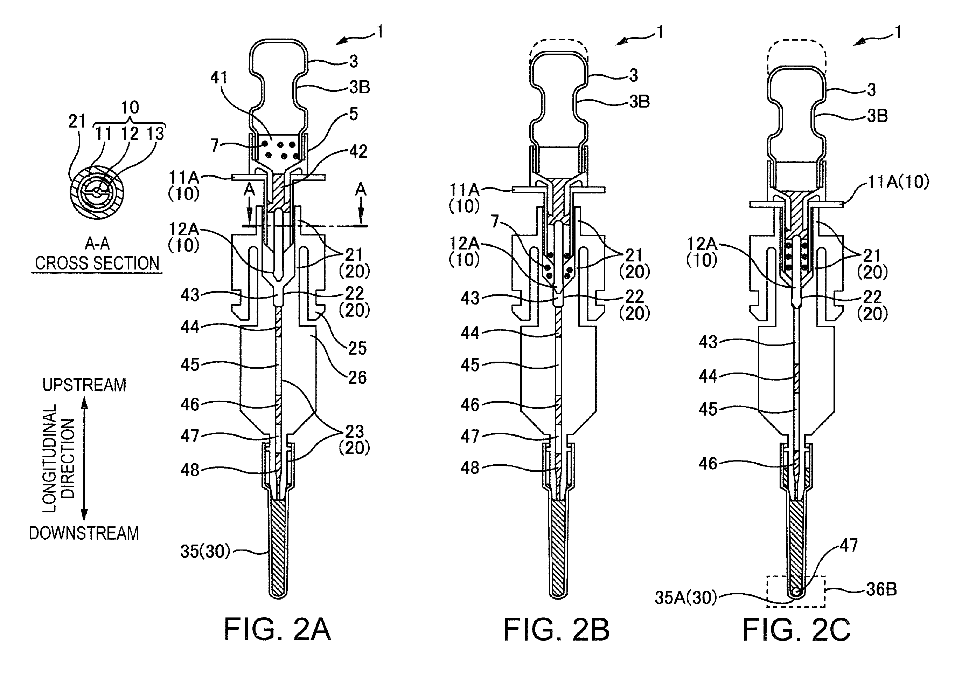

Figs. 1A and 1B are views illustrating a cartridge 1. Figs. 2A to 2C are views illustrating the operation of the cartridge 1. Fig. 2A is a view illustrating the initial state of the cartridge 1. Fig. 2B is a side view when a seal 12A is brought into contact with a lower syringe 22 by pressing a plunger 10 in the state shown in Fig. 2A. Fig. 2C is a view illustrating the cartridge 1 after the plunger 10 is pressed.

The cartridge 1 is a container for performing nucleic acid elution treatment in which nucleic acids are eluted from magnetic beads 7 having nucleic acid bound thereto and also is a container for performing thermal cycling treatment on an eluent 47 for a polymerase reaction.

The nucleic acid extraction treatment is performed in a tank 3 and the nucleic acids are purified while passing through a tube 20. The material for the tube 20 is not particularly limited and examples thereof include glass, resin such as plastic, and metal. Particularly, when transparent glass or resin is selected as the material for the tube 20, the inside of the tube 20 can be observed from the outside, and thus, the use of such a material is more preferable. In addition, when a substance which allows a magnetic force to pass through or a non-magnetic body is used as the material for the tube 20, in a case in which magnetic particles pass through the tube 20, the magnetic particles are easily moved by applying a magnetic force from the outside of the tube 20, and thus, the use of such a substance is preferable. Further, since heaters (eluting heater 65A and high temperature-side heater 65B which will be described later) are disposed in the vicinity of the tube, it is preferable that the material for the tube have a heat resistance of at least 100°C or higher. The material for the tube 20 may be the same as the material for the tank.

The tube 20 has a washing liquid plug 45, an eluent plug 47, and an oil plug. The magnetic beads 7 having nucleic acids bound thereto are attracted to outside magnets and thus the magnets are moved along the tube 20 outside. Then, the magnetic beads 7 move in the tube 20 and reach the eluent plug 47 through the washing liquid plug 45. The nucleic acids bound to the magnetic beads 7 are washed with the washing liquid in the washing liquid plug 45 and eluted in the eluent plug 47. Here, the term of "plug" means a liquid when a predetermined liquid occupies one section in the tube 20. For example, in Figs. 2A to 2C, a liquid that is maintained to have a columnar shape in a capillary 23 is referred to as a "plug". Here, the oil is phase-separated from other solutions (not mixed with other solutions) and thus the plug composed of the oil has a function of preventing the water-soluble plugs disposed on both sides thereof from being mixed with each other. It is preferable that bubbles or other liquids not be present in the plugs or between the plugs. However, as long as the magnetic beads 7 can pass through the plugs, bubbles or other liquids may be present.

The type of the oil is not particularly limited, and mineral oils, silicone oil (2CS silicone oil or the like), and vegetable oil can be used. By using an oil having a higher viscosity, a "wipe-off effect" of the oil at an interface between the plug composed of the oil and the plug immediately upstream thereof can be enhanced when a nucleic acid-binding solid-phase carrier is moved. Accordingly, when the nucleic acid-binding solid-phase carrier is moved to the plug composed of the oil from the plug immediately upstream thereof, this can make it more difficult to carry over water-soluble components attached to the nucleic acid-binding solid-phase carrier into the oil.

The thermal cycling treatment is performed in a PCR container 30 of the cartridge 1 communicating with the tube 20. The PCR container 30 is filled with an oil and the tip end of the tube on the side on which the fifth plug is disposed is preferably brought into contact with the oil in the PCR container 30. The eluent 47 is phase-separated from the oil, and thus when the eluent plug 47 is pushed out from the tube 20 to the PCR container 30, the eluent is in the form of droplets. Further, since the specific gravity of the eluent is greater than the specific gravity of the oil, the eluent 47 in the form of droplets is precipitated. Here, the viscosity of the oil is not particularly limited and is preferably 90 cs or less, more preferably 70 cs or less, still more preferably 50 cs or less, and further still more preferably 30 cs or less. The lower the viscosity is, the more preferable it is. Accordingly, the solution can be smoothly precipitated. The PCR container 30 contains the freeze-dried nucleic acid amplification reaction reagent fixed to the end portion opposite to the side communicating with the tube 20, for example, the bottom of the container. The eluent 47 precipitated in the PCR container 30 is brought into contact with the freeze-dried nucleic acid amplification reaction reagent to dissolve the nucleic acid amplification reaction reagent. On the other hand, a high temperature region 36A and a low temperature region 36B are formed in the PCR container 30 by outside heaters and when an operation of vertically inverting the entire cartridge 1 and heaters is repeated, the eluent 47 in the form of droplets moves alternately between the high temperature region 36A and the low temperature region 36B and the eluent 47 which is a PCR solution is subjected to two-stage temperature treatment.

The material for the PCR container 30 is not particularly limited and examples thereof include glass, resin such as plastic, and metal. In addition, since a high temperature-side heater 65B is disposed in the vicinity thereof, the material for the PCR container 30 preferably has a heat resistance of at least 100°C or higher. When a transparent or translucent material is selected as the material for the PCR container 30, fluorescence measurement (fluorescence intensity measurement) is easy performed, and thus, the use of such a material is preferable. However, the entire region of the PCR container 30 is not necessarily transparent or translucent and at least a portion opposite to a fluorometer 55 (for example, a bottom 35A of the PCR container 30) is preferably transparent or translucent. The material for the PCR container 30 may be the same as the material for the tank 3 or the plunger 10. The preparation method of the freeze-dried nucleic acid amplification reaction reagent in the PCR container 30 is the same as in the first embodiment. The end portion opposite to the side communicating with the tube 20 preferably has a tapered shape and for example, it is preferable that the cross-sectional area decrease toward the tip end. Thus, when the eluent 47 in the form of droplets is precipitated, the eluent can reach the freeze-dried nucleic acid amplification reaction reagent reliably.

The cartridge 1 includes the tank 3 and a cartridge main body 9. In a kit constituting the cartridge 1, an adaptor 5 is prepared with the tank 3 and the cartridge main body 9 in advance. The cartridge 1 is assembled by connecting the tank 3 to the cartridge main body 9 through the adaptor 5. However, it is possible to adopt a configuration in which the tank 3 is directly mounted on the cartridge main body 9.

In the following description of the components of the cartridge 1, as shown in Fig. 2A, a direction along the long cartridge 1 is defined as a "longitudinal direction", a side close to the tank 3 is defined as an "upstream side", and a side close to the PCR container 30 is defined as a "downstream side". The upstream side may be simply referred to as "upper" and the downstream side may be simply referred to as "lower".

### 1. Tank

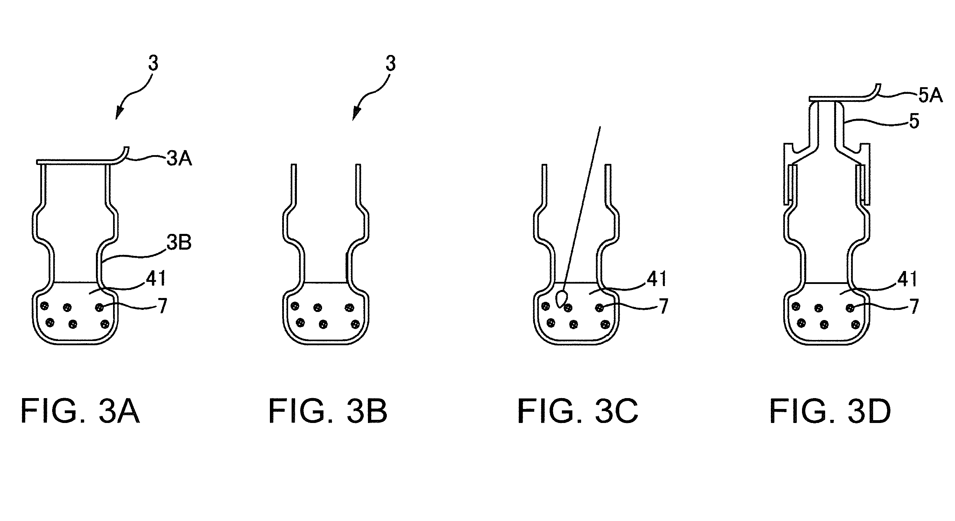

Figs. 3A to 3D are views illustrating the tank 3.

The tank 3 prepared in the kit in advance contains a solution 41 and the magnetic beads 7. A removable cap 3A is mounted on the opening portion of the tank 3 (refer to Fig. 3A). As the solution 41, 5 M guanidine thiocyanate, 2% Triton X-100, and 50 mM Tris-HCl (pH 7.2) are used. An operator removes the cap 3A, opens the opening of the tank 3 (refer to Fig. 3B), and immerses a cotton swab to which viruses are attached in the solution 41 in the tank 3 so as to collect the viruses in the solution 41 (refer to Fig. 3C). When the liquid in the tank 3 is stirred, the tank 3 may be shaken in the state shown in Fig. 3C. However, since the solution 41 easily overflows in this state, it is preferable that an adaptor 5 with a cap 5A be mounted on the opening of the tank 3 as shown in Fig. 3D and then the tank 3 be shaken. Accordingly, the substance in the tank 3 is stirred, the virus particles are dissolved by the solution 41, and the silica with which the magnetic beads 7 are surface-coated adsorbs nucleic acids while the nucleic acids are released. The magnetic bead 7 corresponds to a nucleic acid-binding solid-phase carrier. Thereafter, the operator removes the cap 5A of the adaptor 5 which is mounted on the opening of the tank 3 and mounts the tank 3 on the cartridge main body 9 through the adaptor 5 (refer to Fig. 2A).

The tank 3 is formed of flexible resin and the tank 3 is expandable. When the plunger 10 slides and the state of the plunger is changed from the state shown in Fig. 2A to the state shown in Fig. 2B, the pressure of the liquid in the tube 20 is prevented from increasing excessively due to the expansion of the tank 3 and the liquid in the tube 20 is prevented from being pushed to the downstream side. It is preferable to form a deformation portion 3B in the tank 3 so that the tank 3 easily expands.

A sample from which the nucleic acids are extracted and amplified is not particularly limited to the viruses and cells may be used. The sample is not particularly limited to being deprived from cells and may be derived from a microorganism, a tissue piece or blood of a higher organism or the like.

The solution 41 refers to a liquid for allowing a fine particle (for example, a magnetic particle M) which is a nucleic acid-binding solid-phase carrier to adsorb nucleic acids. The solution 41 is not particularly limited as long as the solution contains a chaotropic substance. The solution may contain a surfactant to destroy cell membranes or modify protein included in cells. The surfactant is not particularly limited as long as the surfactant is generally used for extracting nucleic acids from cells or the like. Specific examples thereof include nonionic surfactants such as Triton-based surfactants such as Triton-X and Tween-based surfactants such as Tween 20, and anionic surfactants such as sodiumN-lauroylsarcosinate (SDS). However, particularly, it is preferable to use a nonionic surfactant in an amount ranging from 0.1% to 2%. Further, the solution preferably contains a reducing agent such as 2-mercaptoethanol or dithiothreitol. The solution 41 may be a buffer solution, but preferably has a neutral pH in a range of 6 to 8. In view of this, specifically, the solution preferably contains a guanidine salt (3 M to 7 M), a nonionic surfactant (0% to 5%), EDTA (0 mM to 0.2 mM), a reducing agent (0 M to 0.2 M), or the like.

The chaotropic substance is not particularly limited as long as the substance generates a chaotropic ion (a monovalent anion having a large ionic radius) in an aqueous solution, has an activity to increase the water solubility of a hydrophobic molecule, and contributes to the adsorption of nucleic acids on the solid-phase carrier. Specific examples thereof include guanidine thiocyanate, guanidine hydrochloride, sodium iodide, potassium iodide, and sodium perchlorate. Among these, guanidine thiocyanate or guanidine hydrochloride having a high protein denaturation activity is preferably used. The used concentration of such a chaotropic substance varies depending on the respective substances, and for example, when guanidine thiocyanate is used, the used concentration thereof is preferably in a range of 3 M to 5.5 M, and when guanidine hydrochloride is used, the used concentration thereof is preferably 5 M or more.

The solution 41 preferably contains alcohol or acetonitrile. In this case, the concentration of alcohol or the like is not particularly limited. The lower limit thereof may be 10% or more, 20% or more, or 30% or more, but 40% or more is most preferable. The upper limit may be 80% or less, 70% or less, or 60% or less but 50% or less is most preferable. The type of alcohol is not particularly limited and examples thereof include methanol, ethanol, and propanol. By adding alcohol or the like to the solution, an effect of adsorbing nucleic acids to a particle or the like is increased and thus a high nucleic acid extraction efficiency can be achieved as a device for extraction.

In addition, a tool for collecting a sample is not particularly limited and instead of the cotton swab, a spatula, a stick, a scraper, or the like may be selected according to applications.

The internal volume of the tank 3 is not particularly limited, and can be set to, for example, 0.1 mL or more and 100 mL or less. The material for the tank 3 is not particularly limited, and examples thereof include glass, resin such as plastic, and metal. Particularly, when transparent glass or resin is selected as the material for the tank, the inside of the tank 3 can be observed from the outside, and thus, the use of such a material is more preferable. It does not matter whether the tank 3 and each tube 20 are integrally formed or detachably formed. When a flexible material such as a rubber, an elastomer, or a polymer is used as a material for the tank 3, it is possible to apply pressure to the inside of the tank 3 by deforming the tank 3 in a state where the cap is mounted on the tank 3. By doing this, the content of the tube 20 can be ejected from the tip end of the tube to the outside from the inside of the tube.

### 2. Cartridge Main Body

The cartridge main body 9 has the plunger 10, the tube 20, and the PCR container 30.

### 2-1. Plunger

Hereinafter, the plunger 10 will be described with reference to Figs. 2A to 2C.

The plunger 10 is a movable plunger for pushing out a liquid from the downstream side of the tube 20 that functions as a syringe. The plunger 10 has a function of pushing a predetermined amount of liquid in the tube 20 from the terminal end of the tube 20 to the PCR container 30. Further, the plunger 10 has a function of mounting the tank 3 through the adaptor 5.

The plunger 10 includes a cylindrical portion 11 and a rod-like portion 12. The cylindrical portion 11 is provided on the side close to the tank 3 (upstream side) and the rod-like portion 12 is provided on the side close to the tube 20 (downstream side). The rod-like portion 12 is supported by two plate-like ribs 13 from the inner wall on the downstream side of the cylindrical portion 11. The downstream side of the rod-like portion 12 is projected from the cylindrical portion 11 to the downstream side.

The cylindrical portion 11 is opened toward the upstream side and the downstream side and the inner wall of the cylindrical portion 11 is a channel of a liquid. The adaptor 5 is fitted to the opening on the upstream side of the cylindrical portion 11 (on the side close to the tank 3). In the plunger 10 of the cartridge main body 9 prepared in advance in the kit, a removable cap may be mounted on the opening on the upstream side of the cylindrical portion 11. The opening on the downstream side of the cylindrical portion 11 is disposed inside an upper syringe 21 of the tube 20. The magnetic beads 7 to be introduced from the opening on the upstream side of the cylindrical portion 11 pass through the inside of the cylindrical portion 11 and both sides of the ribs 13 and come out from the opening on the downstream side of the cylindrical portion 11 and then are introduced to the upper syringe 21 of the tube 20.

The downstream side of the cylindrical portion 11 is fitted to the inner wall of the upper syringe 21 of the tube 20. The cylindrical portion 11 can slide in the longitudinal direction with respect to the upper syringe 21 while being brought into internal contact with the upper syringe 21 of the tube 20.

In the vicinity of the opening on the upstream side of the cylindrical portion 11, a mount 11A for mounting the adaptor 5 is formed. In addition, the mount 11A is also a portion to be pressed when the plunger 10 is pressed. When the mount 11A is pressed, the plunger 10 slides with respect to the tube 20 and the state of the plunger is changed from the state shown in Fig. 2A to the state shown in Fig. 2C. When the plunger 10 moves to the downstream side, the mount 11A is brought into contact with the upper edge of the tube 20 (refer to Fig. 2C). That is, an interval between the mount 11A of the plunger 10 and the upper edge of the tube 20 is a slide length of the plunger 10.

The rod-like portion 12 is disposed inside the upper syringe 21 of the tube 20 in the initial state and is away from the lower syringe 22 (refer to Fig. 2A). When the plunger 10 slides with respect to the tube 20, the rod-like portion 12 is inserted into the lower syringe 22 of the tube 20 and while the rod-like portion 12 slides with respect to the lower syringe 22 in the downstream direction while being brought into internal contact with the lower syringe 22 (refer to Figs. 2B and 2C).

The shape of the cross section of the rod-like portion 12 orthogonal to the longitudinal direction thereof is circular. However, the cross-sectional shape of the rod-like portion 12 can be circular, elliptical, or polygonal and is not particularly limited as long as the cross-sectional shape thereof can be fitted to the inner wall of the lower syringe 22 of the tube 20.

In the end portion on the downstream side of the rod-like portion 12, the seal 12A is formed. When the seal 12A is fitted to the lower syringe 22, the liquid on the downstream side of the tube 20 is prevented from flowing back into the upper syringe 21. Then, when the plunger 10 is pressed from the state shown in Fig. 2B to the state shown in Fig. 2C, the liquid in the tube 20 is pushed out from the downstream side by the amount equivalent to the volume in which the seal 12A slides in the lower syringe 22 in the meantime.

The volume in which the seal 12A slides in the lower syringe 22 (the amount in which the liquid in the tube 20 is pushed out from the downstream side) is larger than a total amount of the eluent plug 47 and the third oil plug 48 in the tube 20. Accordingly, the liquid in the tube 20 can be pushed out so that the eluent 47 does not remain in the tube 20.

The material for the plunger 10 is not particularly limited and examples thereof include glass, resin such as plastic, and metal. In addition, the cylindrical portion 11 and the rod-like portion 12 of the plunger 10 may be integrally formed of the same material or may be formed of different materials. Here, the plunger 10 is formed in such a manner that the cylindrical portion 11 and the rod-like portion 12 are separately molded using resin and the cylindrical portion 11 and the rod-like portion 12 are joined together through the ribs 13.

An oil 42 and a first washing liquid 43 are accommodated in the plunger 10 in advance. The specific gravity of the oil 42 in the plunger 10 is smaller than the specific gravity of the first washing liquid 43, and thus, when the tank 3 is mounted on the cartridge main body 9, the cartridge main body 9 is erected with the mount 11A of the plunger 10 upside and then as shown in Fig. 2A, the oil 42 is disposed between the liquid in the tank 3 and the first washing liquid 43 of the cartridge main body 9. As the oil 42, 2CS silicone oil is used and as the first washing liquid 43, 8 M guanidine hydrochloride and 0.7% Triton X-100 are used.

The first washing liquid 43 may be a liquid which is phase-separated from any of the oil 42 and an oil 44 when being mixed. The first washing liquid 43 is preferably water or an aqueous low salt concentration solution, and when the first washing liquid 43 is an aqueous low salt concentration solution, a buffer solution is preferable. The salt concentration of the aqueous low salt concentration solution is preferably 100 mM or less, more preferably 50 mM or less, and most preferably 10 mM or less. The lower limit of the aqueous low salt concentration solution is not particularly limited and is preferably 0.1 mM or more, more preferably 0.5 mM or more, and most preferably 1 mM or more. In addition, the solution may contain a surfactant such as Triton, Tween, or SDS and the pH thereof is not particularly limited. A salt to be used as a buffer solution is not particularly limited and a salt such as Tris, HEPES, PIPES, or a phosphate is preferably used. Further, the washing liquid preferably contains alcohol in an amount in which adsorption to the nucleic acid carrier, reverse transcription reaction, PCR reaction, or the like is not inhibited. In this case, the concentration of alcohol is not particularly limited and the lower limit thereof may be 50% or more or 60% or more, and is most preferably 70% or more. The upper limit of the concentration of alcohol may be 90% or less or 80% or less, and is most preferably 70% or less. The type of alcohol is not particularly limited and examples thereof include methanol, ethanol, propanol, and acetonitrile. From the viewpoint of washing the nucleic acids and the particle having nucleic acids adsorbed thereon, as long as at least one of the solution and the first washing liquid includes alcohol, the washing effect can be enhanced. When both the solution and the first washing liquid include alcohol, the washing effect can be further enhanced and thus it is preferable that both the solution and the first washing liquid include alcohol.

The first washing liquid 43 may contain a chaotropic agent. For example, when the first washing liquid 43 contains guanidine hydrochloride, the particle or the like can be washed while maintaining or reinforcing the adsorption of the nucleic acids adsorbed on the particle or the like. The concentration of guanidine hydrochloride when the washing liquid contains guanidine hydrochloride may be, for example, 3 mol/L or more and 10 mol/L or less, and preferably 5 mol/L or more and 8 mol/L or less. When the concentration of guanidine hydrochloride is within the above range, other impurities or the like can be washed while the nucleic acids adsorbed on the particle or the like are stably adsorbed.

### 2-2. Tube

Hereinafter, the tube 20 will be described with reference to Figs. 2A to 2C.

The tube 20 has a cylindrical shape capable of allowing a liquid to flow through the tube in the longitudinal direction. The tube 20 has the upper syringe 21, the lower syringe 22, and the capillary 23, and the diameter of each portion is different stepwise.

The upper syringe 21 has a cylindrical shape capable of allowing a liquid to flow through the tube in the longitudinal direction. The cylindrical portion 11 of the plunger 10 is slidably brought into internal contact with the inner wall of the upper syringe 21 and the upper syringe 21 functions as a syringe with respect to the cylindrical portion 11 of the plunger 10.

The lower syringe 22 has a cylindrical shape capable of allowing a liquid to flow through the tube in the longitudinal direction. The seal 12A of the rod-like portion 12 of the plunger 10 is slidably fitted to the inner wall of the lower syringe 22 and the lower syringe 22 functions as a syringe with respect to the rod-like portion 12 of the plunger 10.

The capillary 23 has a small tube capable of allowing a liquid to flow through the tube in the longitudinal direction. The inner diameter of the capillary 23 has a size enough to maintain the plug-shaped liquid and is 1.0 mm here. The diameter of the terminal end of the capillary 23 (end on the downstream side of the tube 20) is smaller than 1.0 mm and is 0.5 mm here. The inner diameter of the terminal end of the capillary 23 is set to be smaller than the diameter of the eluent in the form of droplets (1.5 mm to 2.0 mm), which will be described later. Accordingly, the eluent in the form of droplets when the eluent plug 47 is pushed out from the terminal end of the capillary 23 can avoid adhering to the terminal end of the capillary 23 or flowing back into capillary 23.

The capillary 23 has a hollow interior portion and may have a cylindrical portion capable of allowing a liquid to flow through the tube in the longitudinal direction. The capillary 23 may be bent in the longitudinal direction but is preferably linear. The size and shape of the hollow interior portion of the tube are not particularly limited as long as a liquid can be maintained to have a plug shape in the tube. In addition, the size of the hollow interior portion of the tube or the shape of the cross section perpendicular to the longitudinal direction thereof may vary in the longitudinal direction of the tube.

The shape of the cross section perpendicular to the longitudinal direction of the contour of the tube is also not limited. Further, the thickness (the length from the side surface of the hollow interior portion to the outer surface) of the tube is also not particularly limited. In the case in which the tube has a cylindrical shape, the inner diameter (the diameter of a circle of the cross section perpendicular to the longitudinal direction of the hollow interior portion) thereof can be set to, for example, 0.5 mm or more and 2 mm or less. When the inner diameter of the tube is within this range, the plug composed of a liquid is easily formed within a wide range of the material for the tube and the type of the liquid. The tip end thereof is preferably further narrowed to have a tapered shape and the diameter thereof can be set to 0.2 mm or more and 1 mm or less. Then, it is possible to prevent the eluent 47 having the form of droplets from being adsorbed onto the opening of the capillary 23 and separated from each other in the PCR container 30 by decreasing the inner diameter of the terminal end of the capillary 23 (the diameter of the opening of the capillary 23). However, when the inner diameter of the terminal end of the capillary 23 is too small, a large number of small droplets of the eluent 47 are formed. When the diameters of portions other than the terminal portion of the capillary 23 are narrowed similarly to the terminal portion thereof, the length of the cartridge 1 is increased due to the necessity of ensuring the volume of each plug, and thus, this case is not preferable.

The capillary 23 is internally provided, in the following order from the upstream side, with a first oil plug 44, the washing liquid plug 45, a second oil plug 46, the eluent plug 47, and a third oil plug 48. That is, oil plugs are disposed on both side of water-soluble plugs (the washing liquid plug 45 and the eluent plug 47).

The oil 42 and the washing liquid 43 are accommodated in advance in the upper syringe 21 and the lower syringe 22 on the upstream side of the first oil plug 44 (refer to Fig. 2A). The inner diameters of the upper syringe 21 and the lower syringe 22 are larger than the inner diameter of the capillary 23 and the liquids (the oil 42 and the washing liquid 43) in the upper syringe 21 and the lower syringe 22 cannot be maintained to have a columnar shape like a plug. However, since the first oil plug 44 is maintained to have a plug shape by the capillary 23, the oil constituting the first oil plug 44 is prevented from moving to the upstream side.

The washing liquid plug 45 may be composed of a 5 mM tris-hydrochloric acid buffer solution which is a second washing liquid, but is preferably composed of an acid solution and particularly preferably composed of an acidic aqueous solution. An acid to be contained is not particularly limited and an aqueous solution of citric acid, acetic acid, or glycine hydrochlorate is preferably used. The solution may contain ethylenediaminetetraacetic acid (EDTA) or a surfactant (such as Triton, Tween, or SDS) but is preferably a solution which does not substantially contain a chaotropic substance. The lower limit of the pH thereof is preferably 1 or more, more preferably 2 or more, still more preferably 3 or more, and most preferably 4 or more. The upper limit thereof is preferably 6 or less, more preferably 5 or less, and most preferably 4 or less. By adopting such a first washing liquid, even when the nucleic acids and a particle having nucleic acids adsorbed thereon is brought into contact with a solution including alcohol on the upstream side of the second washing liquid, that is, even when the nucleic acids are extracted with a solution including alcohol or even when the particle having nucleic acids adsorbed thereon is washed with a washing liquid including alcohol, the particle having nucleic acids adsorbed thereon or the like can be efficiently washed with the second washing liquid and the carrying of alcohol to the downstream side, a so-called carry-over of alcohol can be prevented. Thus, it is possible to prevent an enzyme reaction from being inhibited by alcohol.

The washing liquid plug 45 may be composed of plural plugs by being divided by oil plugs. When washing liquid plug 45 is composed of plural plugs, the liquids of the respective plugs may be the same or different. As long as there is at least one plug composed of one washing liquid among the plugs, the liquids of other plugs are not particularly limited. However, it is preferable that all the plugs be composed of a washing liquid. The number of the divided washing liquid plug 45 can be properly set in consideration of, for example, the length of the tube 20, the obj ect to be washed, or the like. At this time, the washing liquid on the side close to the eluent is preferably an acid solution as described in the washing liquid plug 45 and other washing liquids preferably contain alcohol as described in the first washing liquid 43.

The eluent 47 is a liquid for eluting the nucleic acids adsorbed on the nucleic acid-binding solid-phase carrier into a liquid from the carrier and then causing a reverse transcription reaction and polymerase reaction. Accordingly, the eluent 47 may be water or a buffer and may be prepared in advance so that after the nucleic acids are eluted, the eluent 47 is used as a buffer solution used in a reverse transcription reaction and a polymerase reaction as it is. A salt to be used as a buffer solution is not particularly limited as long as the enzyme reaction is not inhibited and a salt such as Tris, HEPES, PIPES, or a phosphate is preferably used. Further, the eluent may contain a reverse transcriptase, dNTP, and a primer for the reverse transcriptase (oligonucleotide) for the reverse transcription reaction. Further, the eluent preferably contains bovine serum albumin (BSA) or gelatin as a preventive agent for reaction inhibition. A solvent is preferably water but is preferably a solution which does not substantially contain an organic solvent such as ethanol or isopropyl alcohol and chaotropic substances.

The concentrations of the dNTP and the salt contained in the eluent may be set to concentrations suitable for an enzyme to be used finally in consideration of the concentrations of the dNTP and the salt in the freeze-dried nucleic acid amplification reaction reagent. The concentration of the dNTP may be set to generally 10 µM to 1000 µM, and preferably 100 µM to 500 µM, the concentration of Mg²⁺ may be set to 1 mM to 100 mM, and preferably 5 mM to 10 mM, and the concentration of Cl⁻ may be set to 1 mM to 2000 mM, and preferably 200 mM to 700 mM. The total ion concentration is not particularly limited, but may be higher than 50 mM, and is preferably higher than 100 mM, more preferably higher than 120 mM, still more preferably higher than 150 mM, and further still more preferably higher than 200 mM. The upper limit thereof is preferably 500 mM or less, more preferably 300 mM or less, and still more preferably 200 mM or less. Each oligonucleotide as the primer is used in an amount of 0.1 µM to 10 µM, and preferably 0.1 µM to 1 µM. When the concentration of BSA or gelatin is 1 mg/mL or less, the preventive effect on reaction inhibition is small, and when the concentration thereof is 10 mg/mL or more, it may inhibit the reverse transcription reaction or the subsequent enzymatic reaction, and therefore, the concentration thereof is preferably from 1 mg/mL to 10 mg/mL. In the case of using gelatin, the gelatin may be derived from, for example, cattle skin, pig skin, or cattle bone, and the origin thereof is not particularly limited thereto. When the gelatin is sparsely soluble, the gelatin may be heated to facilitate dissolution.

The volume of the eluent plug 47 is not particularly limited and can be appropriately set by using the amount of the particle having nucleic acids adsorbed thereon or the like as an index. For example, when the volume of the particle or the like is 0.5 µL, it is sufficient that the volume of the eluent plug 47 is 0.5 µL or more, and it is set to preferably 0.8 µL or more and 5 µL or less, more preferably 1 µL or more and 3 µL or less. In the case where the volume of the eluent plug 47 is within these ranges, for example, even when the volume of the nucleic acid-binding solid-phase carrier is set to 0.5 µL, the nucleic acids can be sufficiently eluted from the carrier.

The downstream portion of the capillary 23 is inserted into the PCR container 30. By doing this, the eluent plug 47 in the tube 20 is pushed out from the tube 20 and then the eluent 47 can be introduced into the PCR container 30.

An upper sealing portion is formed by brining the annular projection on the outer wall of the capillary 23 into contact with the inner wall of the PCR container 30. In addition, a lower sealing portion is formed by bringing the outer wall of the capillary 23 on the downstream side of the upper sealing portion into contact with the inner wall of the PCR container 30. The upper sealing portion and the lower sealing portion will be described later.

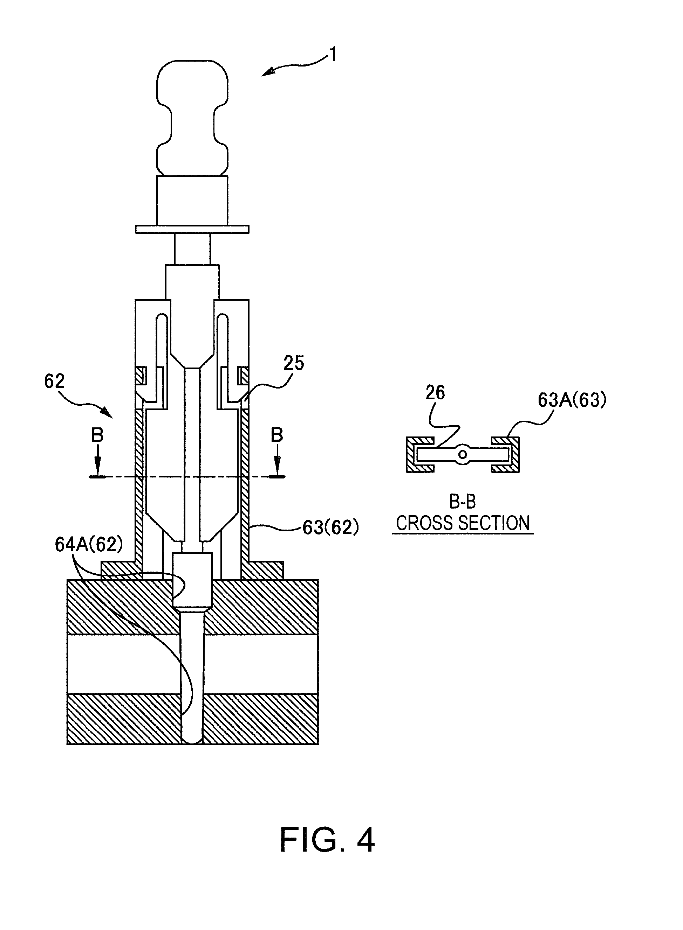

The tube 20 further includes a fixing claw 25 and a guide plate 26. Fig. 4 is a view illustrating the fixing claw 25 and the guide plate 26 and a mounting portion 62.

The fixing claw 25 is a member for fixing the cartridge 1 to the mounting portion 62. When the cartridge 1 is inserted into the mounting portion 62 until the fixing claw 25 is hooked, the cartridge 1 is fixed to the mounting portion 62 in a normal position. In other words, when the cartridge 1 is disposed at an abnormal position with respect to the mounting portion 62, the fixing claw 25 is not hooked to the mounting portion 62.

The guide plate 26 is a member for guiding the cartridge 1 when the cartridge 1 is mounted on the mounting portion 62 of the PCR apparatus 50. A guide rail 63A is formed in the mounting portion 62 of the PCR apparatus 50, and the cartridge 1 is inserted and fixed to the mounting portion 62 while being guided by the guide plate 26 of the tube 20 along the guide rail 63A. The cartridge 1 has a long shape but the cartridge 1 is inserted into the mounting portion 62 while being guided by the guide plate 26 and thus the cartridge 1 can be easily fixed to the mounting portion 62 in a normal position.

The fixing claw 25 and the guide plate 26 are plate-like members projecting from the right and left sides of the capillary 23. When the magnetic beads 7 in the tube 20 are moved by a magnet, the magnet approaches from a direction perpendicular to the plat-like fixing claw 25 and guide plate 26. Thus, a distance between the magnet and the magnetic beads 7 in the tube 20 can be reduced. However, as long as the distance between the magnet and the magnetic beads 7 in the tube 20 can be reduced, the fixing claw 25 and the guide plate 26 may have any shape.

### 2-3. PCR Container

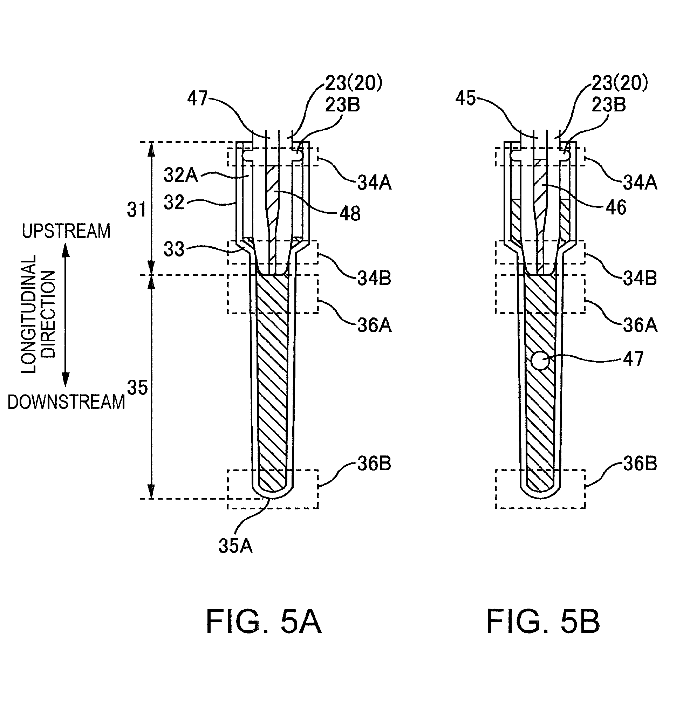

Figs. 5A and 5B are views illustrating the vicinity of the PCR container 30. Fig. 5A is a view illustrating an initial state. Fig. 5B is a view illustrating a state after the plunger 10 is pressed. Hereinafter, the PCR container 30 will be described with reference to Figs, 2A to 2C.

The PCR container 30 is a container which receives the liquid pressed out from the tube 20 and is also a container which contains the freeze-dried nucleic acid amplification reaction reagent and also is a container which accommodates the eluent 47 at the time of thermal cycling treatment. The PCR container corresponds to the container for nucleic acid amplification reaction and the configuration thereof is the same as that of the first embodiment.

The PCR container 30 includes a seal forming portion 31 and a flow path forming portion 35. The seal forming portion 31 is a portion into which the tube 20 is inserted and is a portion which is provided for preventing an oil overflowing from the flow path forming portion 35 from leaking outside. The flow path forming portion 35 is a portion provided on the downstream side of the seal forming portion 31 and is a portion for forming a flow path in which the eluent 47 in the form of droplets moves. The PCR container 30 is fixed to the tube 20 in two portions of an upper sealing portion 34A and a lower sealing portion 34B in the seal forming portion 31.

The seal forming portion 31 includes an oil receiving portion 32 and a step portion 33.

The oil receiving portion 32 is a cylindrical portion and functions as a receiver which receives the oil overflowing from the flow path forming portion 35. There is an interval between the inner wall of the oil receiving portion 32 and the outer wall of the capillary 23 of the tube 20 and the interval is an oil receiving space 32A which receives the oil overflowing from the flow path forming portion 35. The volume of the oil receiving space 32A is larger than the volume in which the seal 12A of the plunger 10 slides in the lower syringe 22 of the tube 20.

The upper sealing portion 34A is formed by bringing the inner wall on the upstream side of the oil receiving portion 32 into contact with the annular projection of the tube 20. The upper sealing portion 34A functions as a seal which prevents the oil of the oil receiving space 32A from leaking outside while allowing the air to flow. In the upper sealing portion 34A, a vent hole is formed with a size in which the oil does not leak out due to the surface tension of the oil. The vent hole of the upper sealing portion 34A may be an interval between the projection of the tube 20 and the inner wall of the oil receiving portion 32, or may be a hole, a groove, or a notch formed in the projection of the tube 20. Further, the upper sealing portion 34A may be formed of an oil absorbing member which absorbs an oil.

The step portion 33 is a portion which is provided on the downstream side of the oil receiving portion 32 and has a step. The inner diameter on the downstream side of the step portion 33 is smaller than the inner diameter of the oil receiving portion 32. The inner wall of the step portion 33 is brought in contact with the outer wall on the downstream side of the capillary 23 of the tube 20. The lower sealing portion 34B is formed by brining the inner wall of the step portion 33 into contact with the outer wall of the tube 20. The lower sealing portion 34B functions as a seal which resists to the flowing of the oil while allowing the oil in the flow path forming portion 35 to flow into the oil receiving portion 32A. The pressure of the flow path forming portion 35 becomes higher than the outside pressure due to pressure loss in the lower sealing portion 34B and thus even when the liquid in the flow path forming portion 35 is heated at the time of thermal cycling treatment, bubbles are not easily generated in the liquid in the flow path forming portion 35.

The flow path forming portion 35 is a tubular portion and is a container for forming the flow path in which the eluent 47 in the form of droplets moves. The flow path forming portion 35 is filled with an oil. The upstream side of the flow path forming portion 35 is closed by the terminal end of the tube 20, and the terminal end of the tube 20 is opened toward the flow path forming portion 35. The inner diameter of the flow path forming portion 35 is larger than the inner diameter of the capillary 23 of the tube 20 and is larger than the outer diameter of the liquid when the liquid of the volume of the eluent plug 47 is formed into a spherical shape. The inner wall of the flow path forming portion 35 preferably has water repellency to a degree to which the water-soluble eluent 47 does not adhere.

The upstream side of the flow path forming portion 35 is heated to a relatively high temperature (for example, about 95°C) by the high temperature-side heater 65B outside to form a high temperature region 36A. The downstream side of the flow path forming portion 35 is heated to a relatively low temperature (for example, about 60°C) by an outside low temperature-side heater 65C to form a low temperature region 36B. The bottom 35A of the PCR container 30 (end portion on the downstream side) is included in the low temperature region 36B. Thus, a temperature gradient is generated in the liquid in the flow path forming portion 35.

As shown in Fig. 5A, the flow path forming portion 35 of the PCR container 30 is filled with an oil in the initial state. An interface of the oil is disposed on a relatively downstream side of the oil receiving portion 32A. The volume of the oil receiving portion 32A on the upstream side of the interface of the oil is larger than the volume in which the seal 12A of the plunger 10 slides in the lower syringe 22 of the tube 20.

As shown in Fig. 5B, when the plunger 10 is pressed, the liquid in the tube 20 is pushed out to the flow path forming portion 35. The flow path forming portion 35 is filled with an oil in advance and the liquid in the tube 20 is pushed out, and thus, gas does not flow into the flow path forming portion 35.

When the plunger 10 is pressed, first, the third oil plug 48 in the tube 20 flows into the flow path forming portion 35 and the oil flows from the flow path forming portion 35 to the oil receiving portion 32A by the amount of the third oil plug inflow, and the interface of the oil in the oil receiving portion 32A rises. At this time, due to pressure loss in the lower sealing portion 34B, the pressure of the liquid in the flow path forming portion 35 increases. After the third oil plug 48 is pushed out from the tube 20, the eluent plug 47 flows into the flow path forming portion 35 from the tube 20. Since the inner diameter of the flow path forming portion 35 is larger than the inner diameter of the capillary 23, the eluent 47 having a plug shape (columnar shape) in the tube 20 is formed in the form of droplets in the oil in the flow path forming portion 35. Since the volume of the oil receiving portion 32A on the upstream side of the interface of the oil in the initial state is larger than the volume in which the syringe 12A of the plunger 10 slides in the lower syringe 22 of the tube 20, the oil does not overflow from the oil receiving portion 32A. The eluent 47 in the form of droplets dissolves the freeze-dried nucleic acid amplification reaction reagent in the PCR container 30 to produce a RT-PCR reaction solution or a PCR reaction solution.

### PCR Apparatus 50

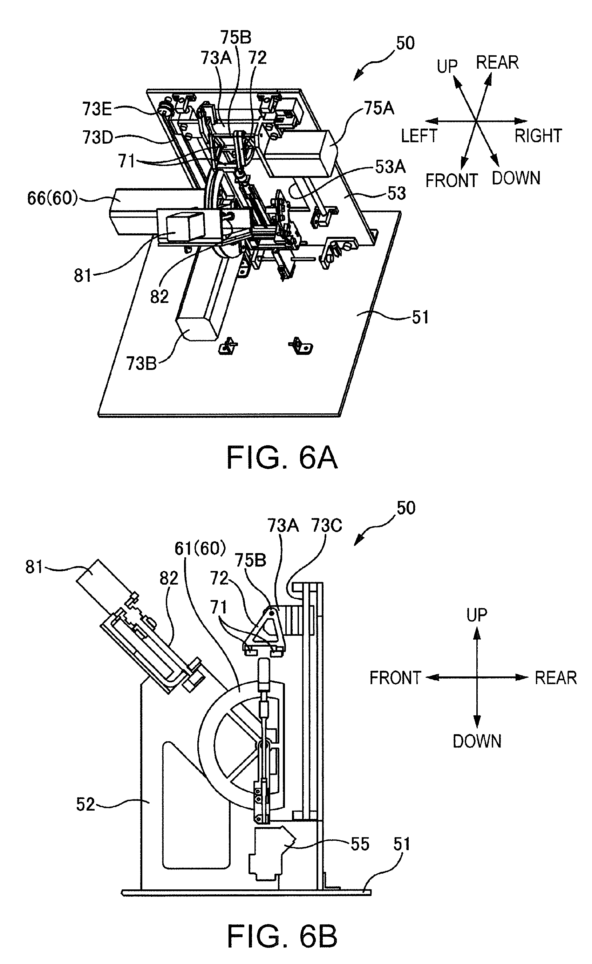

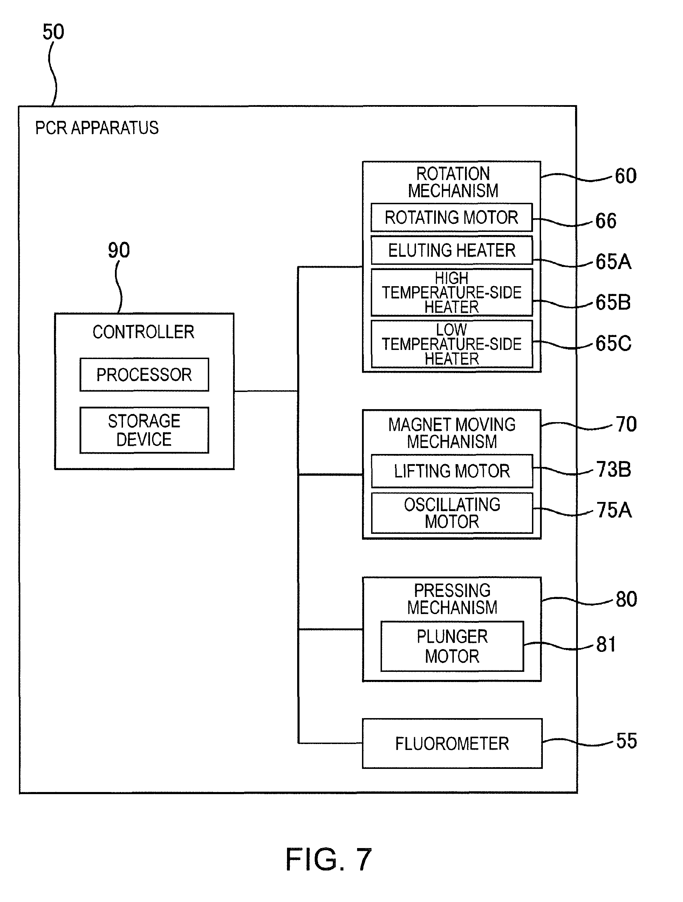

Fig. 6A is a perspective view illustrating the inner configuration of a PCR apparatus 50. Fig. 6B is a side view illustrating the main configuration of the PCR apparatus 50. Fig. 7 is a block diagram illustrating the PCR apparatus 50. The PCR apparatus 50 is an apparatus for performing nucleic acid elution treatment and thermal cycling treatment using the cartridge 1.

In the following description of the PCR apparatus 50, as shown in the drawings, the terms of up and down, front and rear, and left and right will be defined. That is, the vertical direction when a base 51 of the PCR apparatus 50 is arranged horizontally is defined as an "up and down direction" and defined as "up" and "down" along the gravity direction. In addition, the axial direction of the rotation axis of the cartridge 1 is defined as a "left and right direction", and a direction perpendicular to the up and down direction and the left and right direction is defined as a "front and rear direction". When viewed from the rotation axis of the cartridge 1, the side close to a cartridge insertion port 53A is defined as "rear" and the opposite side is defined as "front". When viewed from the front side, the right side in the left and right direction is defined as "right" and the left side is defined as "left".

The PCR apparatus 50 includes a rotation mechanism 60, a magnet moving mechanism 70, a pressing mechanism 80, a fluorometer 55, and a controller 90.

### 1. Rotation Mechanism 60

The rotation mechanism 60 is a mechanism which rotates the cartridge 1 and heaters. When the rotation mechanism 60 vertically inverts the cartridge 1 and heaters, the eluent 47 in the form of droplets moves in the flow path forming portion 35 of the PCR container 30 and thermal cycling treatment is performed.

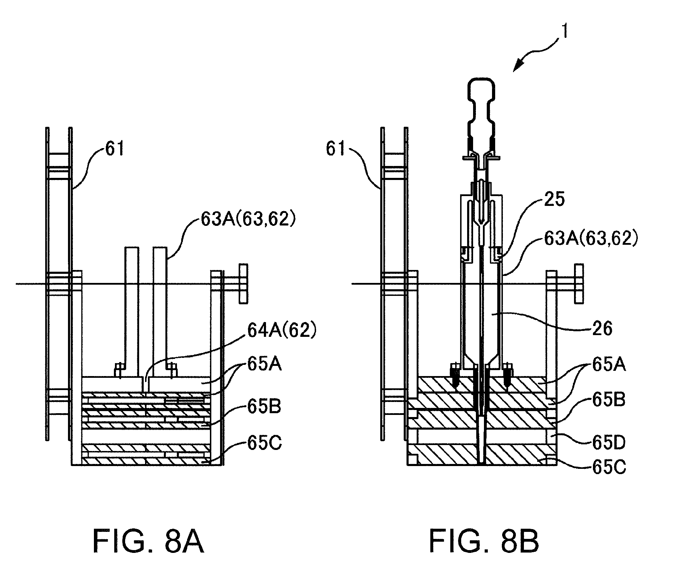

The rotation mechanism 60 includes a rotary body 61 and a rotating motor 66. Fig. 8A is a view illustrating the rotary body 61. Fig. 8B is a view illustrating a state in which the cartridge 1 is mounted on the mounting portion 62 of the rotary body 61.

The rotary body 61 is a member which is rotatable around the rotation axis. The rotation axis of the rotary body 61 is supported by a supporting base 52 that is fixed to the base 51. The rotary body 61 is provided with the mounting portion 62 on which the cartridge 1 is mounted and heaters (eluting heater 65A, high temperature-side heater 65B, and low temperature-side heater 65C). When the rotary body 61 rotates, the cartridge 1 can be inverted vertically while the positional relationship between the cartridge 1 and the heaters is maintained. The rotating motor 66 is a power source for rotating the rotary body 61. The rotating motor 66 rotates the rotary body 61 at a predetermined position in response to the instruction from the controller 90. A transmission mechanism such as a gear may be interposed between the rotating motor 66 and the rotary body 61.

The rotation axis of the rotary body 61 is positioned at a position closer to the tube 20 than to the PCR container 30 of the cartridge 1. In other words, the height of the rotation axis of the rotary body 61 is positioned at the height of the tube 20 of the cartridge 1 mounted on the mounting portion 62. The tube 20 is longer than the PCR container 30, and thus, if the center of the PCR container 30 is set to the rotation axis (if the height of the rotation axis of the rotary body 61 is positioned at the height of the PCR container), the size of the rotary body 61 is increased.

The mounting portion 62 is a portion on which the cartridge 1 is mounted. The mounting portion 62 includes a fixing portion 63 in which a notch is formed. Further, an insertion hole 64A formed in the heaters (eluting heater 65A, high temperature-side heater 65B, and low temperature-side heater 65C) also functions as the mounting portion 62. When the fixing claw 25 of the cartridge 1 is hooked to the notch of the fixing portion 63 in a state in which the PCR container 30 is inserted into the insertion hole 64A, the cartridge 1 is mounted on the rotary body 61 (refer to Fig. 4). Here, a part of the heaters also functions as the mounting portion 62. However, the mounting portion 62 and the heaters may be independent. Further, the mounting portion 62 is indirectly fixed to the rotary body 61 through the eluting heater 65A. However, the mounting portion maybe directly fixed to the rotary body 61. In addition, the number of the cartridge 1 that can be mounted on the mounting portion 62 is not limited to 1 and plural cartridges may be mounted.

The fixing portion 63 of the mounting portion 62 functions as a tube fixing portion for fixing the tube 20 of the cartridge 1 and the insertion hole 64A functions as a PCR container fixing portion for fixing the PCR container 30. Thus, the long cartridge 1 formed of the tube 20 and the PCR container 30 is stably fixed to the mounting portion 62.

The guide rail 63A is formed in the fixing portion 63 in the up and down direction (refer to Fig. 4). The guide rail 63A guides the guide plate into the insertion direction while binding the guide plate 26 of the cartridge 1 in the front and rear direction. The cartridge 1 is inserted into the mounting portion 62 while the guide plate 26 is guided by the guide rail 63A, and thus the PCR container 30 of the cartridge 1 is guided to the insertion hole 64A and the cartridge 1 is fixed to the mounting portion 62 in a normal position.

The PCR apparatus 50 includes the eluting heater 65A, the high temperature-side heater 65B as a heater for PCR, and the low temperature-side heater 65C. Each heater is formed of a heat generation source (not shown) and a heat block. The heat generation source is, for example, a cartridge heater, and is inserted into the heat block. The heat block is, for example, a metal such as aluminum having a high thermal conductivity and heats the liquid in the cartridge 1 with the heat from the heat generation source while suppressing uneven heating. Further, the heat block is preferably non-magnetic so that magnets 71 which move the magnetic beads 7 are not adsorbed.

The eluting heater 65A is a heater which heats the eluent plug 47 of the cartridge 1. The eluting heater 65A is opposite to the eluent plug 47 of the tube 20 when the cartridge 1 is fixed in a normal position. For example, when the eluting heater 65A heats the eluent plug 47 to about 50°C, release of the nucleic acids from the magnetic beads is promoted.

The high temperature-side heater 65B is a heater which heats the upstream side of the flow path forming portion 35 of the PCR container 30. The high temperature-side heater 65B is opposite to the upstream side of the flow path forming portion 35 of the PCR container 30 (high temperature region 36A) when the cartridge 1 is fixed in a normal position. For example, the high temperature-side heater 65B heats the liquid on the upstream side of the flow path forming portion 35 of the PCR container 30 to about 90°C to 100°C.

The low temperature-side heater 65C is a heater which heats the bottom 35A of the flow path forming portion 35 of the PCR container 30. The low temperature-side heater 65C is opposite to the downstream side of the flow path forming portion 35 of the PCR container 30 (low temperature region 36B) when the cartridge 1 is fixed in a normal position. For example, the low temperature-side heater 65C heats the liquid in the low temperature region 36B of the PCR container 30 to about 50°C to 75°C.

A spacer 65D is disposed between the high temperature-side heater 65B and the low temperature-side heater 65C. The spacer 65D suppresses thermal conduction between the high temperature-side heater 65B and the low temperature-side heater 65C. In addition, the spacer 65D is used for accurately determining a distance between the high temperature-side heater 65B and the low temperature-side heater 65C. Thus, a temperature gradient is generated in the liquid in the flow path forming portion 35 of the PCR container 30 by the high temperature-side heater 65B and the low temperature-side heater 65C.

In the heat blocks forming the eluting heater 65A, the high temperature-side heater 65B, and the low temperature-side heater 65C respectively, through holes forming the insertion hole 64A are formed respectively. The outer wall of the bottom 35A of the PCR container 30 is exposed from the lower opening of the insertion hole 64A of the low temperature-side heater 65C. The fluorometer 55 measures the brightness of the eluent 47 from the lower opening of the insertion hole 64A.

Temperature control devices are provided respectively in the high temperature-side heater 65B and the low temperature-side heater 65C and the temperature of each heater can be set to a temperature suitable for a polymerase reaction.

### 2. Magnet Moving Mechanism 70

The magnet moving mechanism 70 is a mechanism which moves the magnets 71. The magnet moving mechanism 70 moves the magnetic beads 7 in the cartridge 1 by moving the magnets 71 while allowing the magnetic beads 7 in the cartridge 1 to be attracted to the magnets 71. The magnet moving mechanism 70 includes a pair of magnets 71, a lifting mechanism 73, and an oscillation mechanism 75.

The magnets 71 are members which attract the magnetic beads 7. As the magnet 71, a permanent magnet, an electromagnet, or the like can be used. However, a permanent magnet is used here in view of not causing heat generation or the like. The pair of magnets 71 are held by an arm 72 so that the magnets are opposite to each other in the front and rear direction and the positions thereof is almost the same in the up and down direction. Each magnet 71 can be disposed to be opposite to each other from the front side or the rear side of the cartridge 1 mounted on the mounting portion 62. The cartridge 1 mounted on the mounting portion 62 can be interposed between the pair of magnets 71 from the front and rear direction. When the magnets 71 are opposite to each other from a direction orthogonal to the direction in which the fixing claw 25 or the guide plate 26 of the cartridge 1 is provided (herein, the left and right direction) (herein, the front and rear direction), a distance between the magnetic beads 7 in the cartridge 1 and the magnets 71 can be reduced.

The lifting mechanism 73 is a mechanism which moves the magnets 71 in the up and down direction. The magnets 71 attract the magnetic beads 7 and thus as long as the magnets 71 are moved in the up and down direction according to the movement of the magnetic beads 7, the magnetic beads 7 in the cartridge 1 can be attracted in the up and down direction.

The lifting mechanism 73 includes a carriage 73A which moves in the up and down direction and a lifting motor 73B. The carriage 73A is member which can move in the up and down direction and is guided by a carriage guide 73C provided on a side wall 53 of the cartridge insertion port 53A so as to be movable in the up and down direction. The arm 72 which holds the pair of magnets 71 is mounted on the carriage 73A, and thus, when the carriage 73A moves in the up and down direction, the magnets 71 move in the up and down direction. The lifting motor 73B is a power source for moving the carriage 73A in the up and down direction. The lifting motor 73B moves the carriage 73A to a predetermined position in the up and down direction according to the instruction from the controller 90. The lifting motor 73B moves the carriage 73A in the up and down direction using a belt 73D and a pulley 73E. However, the lifting motor may move the carriage 73A in the up and down direction using other transfer mechanisms.

When the carriage 73A is positioned at the height position (retractable position), the magnets 71 are positioned on the upper side of the cartridge 1. When the carriage 73A is at the retractable position, the lifting mechanism 73 is not brought into contact with the cartridge 1 even in a case in which the cartridge 1 rotates. In addition, the lifting mechanism 73 can lower the position of the carriage 73A to a position in which the magnets 71 are opposite to the reaction plug. Accordingly, the lifting mechanism 73 can move the magnets 71 so that the magnetic beads 7 in the tank 3 are moved to the position of the reaction plug.

The oscillation mechanism 75 is a mechanism which oscillates the pair of magnets 71 in the front and rear direction. When the pair of magnets 71 are oscillated in the front and rear direction, the interval between each magnet 71 and the cartridge 1 is changed alternately. The magnetic beads 7 are attracted to a closer one of the magnets 71 and thus the magnetic beads 7 in the cartridge 1 move in the front and rear direction by oscillating the pair of magnets 71 in the front and rear direction.

The oscillation mechanism 75 includes an oscillating motor 75A and a gear. The oscillating motor 75A and the gear are provided in the carriage 73A and can move with the carriage 73A in the up and down direction. When the power of the oscillating motor 75A is transmitted to the arm 72 through the gear, the arm 72 which holds the magnets 71 rotates around an oscillating rotation axis 75B with respect to the carriage 73A. The oscillation mechanism 75 oscillates the magnets 71 within a range in which the magnets 71 are not brought into contact with the cartridge 1 so as to prevent the magnets 71 from damaging the cartridge 1 by the contact with the cartridge 1.

The oscillating rotation axis 75B is the rotation axis of the arm 72. The oscillating rotation axis 75B is provided to be parallel in the left and right direction so that the magnets 71 can be oscillated in the front and rear direction. When the oscillating rotation axis 75B is viewed from the right or the left side, the oscillating rotation axis 75B is disposed to be shifted from the cartridge 1 to the front side or the rear side. Thus, when the carriage 73A moves downward, the contact between the cartridge 1 and the arm 72 can be avoided. As long as the magnets 71 can be oscillated in the front and rear direction, the oscillating rotation axis 75B may be an axis parallel in the up and down direction.

### 3. Pressing Mechanism 80

The pressing mechanism 80 is a mechanism which presses the plunger 10 of the cartridge 1. When the plunger 10 is pressed by the pressing mechanism 80, the eluent plug 47 and the oil plug in the cartridge 1 is pushed out to the PCR container 30, and the eluent 47 is formed into droplets in the oil in the PCR container 30.

The pressing mechanism 80 includes a plunger motor 81 and a rod 82. The plunger motor 81 is a power source for moving the rod 82. The rod 82 is a member which presses the mount 11A of the plunger 10 of the cartridge 1. The reason for pressing not the tank 3 of the cartridge 1 but the mount 11A is that the tank 3 is composed of an expandable and flexible resin. When the tank 3 is not deformed, the plunger 10 may be pressed by the pressing mechanism 80 pressing the tank 3.

The direction in which the rod 82 presses the plunger 10 is not the up and down direction and is inclined at 45 degrees in the up and down direction. Therefore, when the plunger 10 is pressed by the pressing mechanism 80, the PCR apparatus 50 moves the rod 82 while matching the longitudinal direction of the cartridge 1 with the movement direction of the rod 82 by rotating the rotary body 61 by 45 degrees. Since the direction in which the rod 82 presses the plunger 10 is inclined at 45 degrees in the up and down direction, the pressing mechanism 80 can be easily disposed not to interfere with the lifting mechanism 73. In addition, since the direction in which the rod 82 presses the plunger 10 is inclined at 45 degrees in the up and down direction, the size of the PCR apparatus 50 in the up and down direction can be reduced.

### 4. Fluorometer 55

The fluorometer 55 includes an excitation light source which emits excitation light to the eluent 47 in the PCR container 30 and a fluorophotometer which measures the intensity of the fluorescence emitted from the eluent 47. The fluorometer 55 is dispose on the lower side of the rotary body 61 so as to be opposite to the bottom 35A of the PCR container 30 of the cartridge 1. The fluorometer 55 measures the intensity of the fluorescence emitted from the eluent 47 on the bottom 35A of the PCR container 30 from the lower opening of the insertion hole 64A of the low temperature-side heater 65C.

### 5. Controller 90

The controller 90 is a control device which controls the PCR apparatus 50. The controller 90 includes, for example, a processor such as a CPU and a storage device such as a ROM or RAM. In the storage device, various programs and data are stored. In addition, the storage device provides an area in which a program is deployed. When the processor executes a program stored in the storage device, various processes are realized.

For example, the controller 90 controls the rotating motor 66 to rotate the rotary body 61 to a predetermined rotational position. A rotational position sensor (not shown) is provided in the rotation mechanism 60 and the controller 90 controls the rotating motor 66 to be driven or stop according to the detection result of the rotational position sensor.

In addition, the controller 90 controls each of the heaters (eluting heater 65A, high temperature-side heater 65B, and low temperature-side heater 65C) to generate heat. The heat blocks forming the heaters are provided with temperature sensors (not shown) and the controller 90 controls on-off of the cartridge heater according to the detection results of the temperature sensors.

Further, the controller 90 controls the lifting motor 73B to move the magnets 71 in the up and down direction. A position sensor (not shown) which detects the position of the carriage 73A is provided in the PCR apparatus 50, and the controller 90 controls the lifting motor 73B to be driven or stop according to the detection result of the position sensor.

In addition, the controller 90 controls the oscillating motor 75A to oscillate the magnets 71 in the front and rear direction. A position sensor which detects the position of the arm 72 which holds the magnets 71 is provided in the PCR apparatus 50 and the controller 90 controls the oscillating motor 75A to be driven or stop according to the detection result of the position sensor.

Further, the controller 90 controls the fluorometer 55 to measure the intensity of the fluorescence of the eluent 47 in the PCR container 30. The controller 90 controls the fluorometer 55 to perform measurement when the fluorometer 55 is opposite to the bottom 35A of the PCR container 30 of the cartridge 1. The measurement result is stored in the storage device.

### Operation Description

### 1. Mounting Operation of Cartridge 1

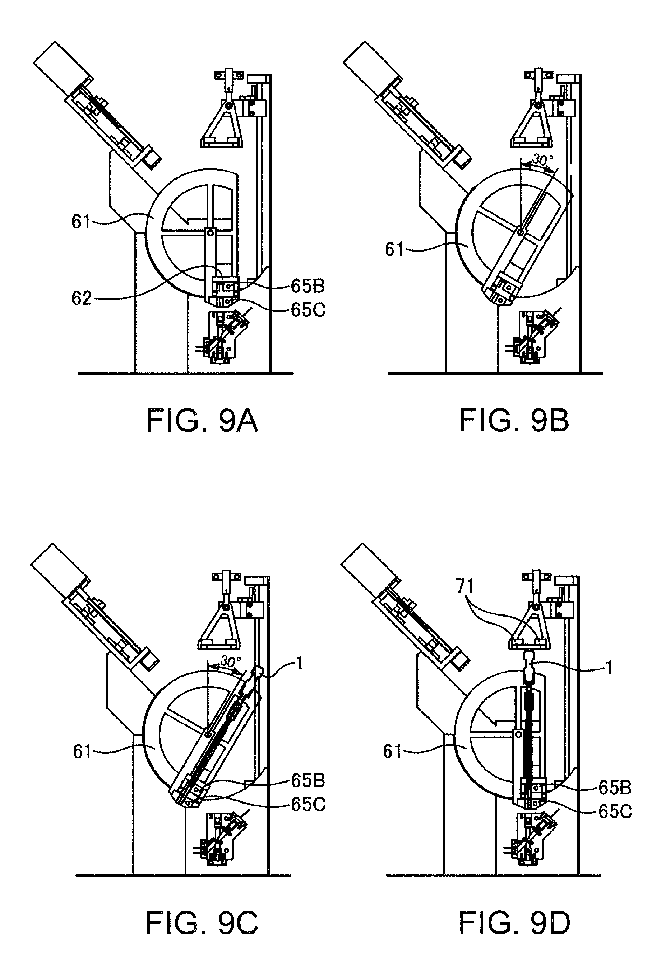

Figs. 9A to 9D are views illustrating states of the PCR apparatus 50 when the cartridge 1 is mounted. Fig. 9A is a view illustrating an initial state before the cartridge 1 is mounted. Fig. 9B is a view illustrating a standby state. Fig. 9C is a view illustrating a state immediately after the cartridge 1 is mounted. Fig. 9D is a view illustrating the initial state when the cartridge 1 is mounted.

As shown in Fig. 9A, the mounting direction of the mounting portion 62 is the up and down direction in an initial state before the cartridge 1 is mounted. In the following description, the rotational position of the rotary body 61 in this state is set as a reference (0 degree), a counterclockwise direction when viewed from the right side is set as a positive direction to indicate the rotational position of the rotary body 61.

As shown in Fig. 9B, the controller 90 drives the rotating motor 66 to rotate the rotary body 61 by -30 degrees. In this state, an operator inserts the cartridge 1 into the mounting portion 62 from the cartridge insertion port 53A. At this time, the cartridge 1 is inserted into the mounting portion 62 while the guide plate 26 is guided by the guide rail 63A, and thus, the PCR container 30 of the cartridge 1 is guided to the insertion hole 64A of the mounting portion 62. The operator inserts the cartridge 1 into the mounting portion until the fixing claw 25 of the cartridge 1 is hooked to the notch of the fixing portion 63. Thus, the cartridge 1 is fixed to the mounting portion 62 in a normal position. If the PCR container 30 is not inserted into the insertion hole 64A and the cartridge 1 is positioned in an abnormal position with respect to the mounting portion 62, the fixing claw 25 of the cartridge 1 is not hooked to the notch of the fixing portion 63 and thus the operator can recognize that the cartridge 1 is positioned in an abnormal position.

As shown in Fig. 9C, when the cartridge 1 is fixed to the mounting portion 62 in a normal position, the eluent plug 47 of the tube 20 is opposite to the eluting heater 65A, the upstream side of the flow path forming portion 35 of the PCR container 30 (high temperature region 36A) is opposite to the high temperature-side heater 65B, and the downstream side of the flow path forming portion 35 of the PCR container 30 (low temperature region 36B) is opposite to the low temperature-side heater 65C. The mounting portion 62 and the heaters are provided in the rotary body 61 and thus even when the rotary body 61 rotates, the positional relationship between the cartridge 1 and the heaters is maintained as it is.

After the cartridge 1 is mounted on the mounting portion 62, as shown in Fig. 9D, the controller 90 controls the rotary body 61 to rotate by 30 degrees to return the position of the rotary body 61 to the reference. The controller 90 may detect the mounting of the cartridge 1 on the mounting portion 62 by a sensor (not shown) or may detect the mounting of the cartridge by an input operation by the operator.

### 2. Nucleic Acid Elution Treatment

### • Up-and-down Movement of Magnets 71

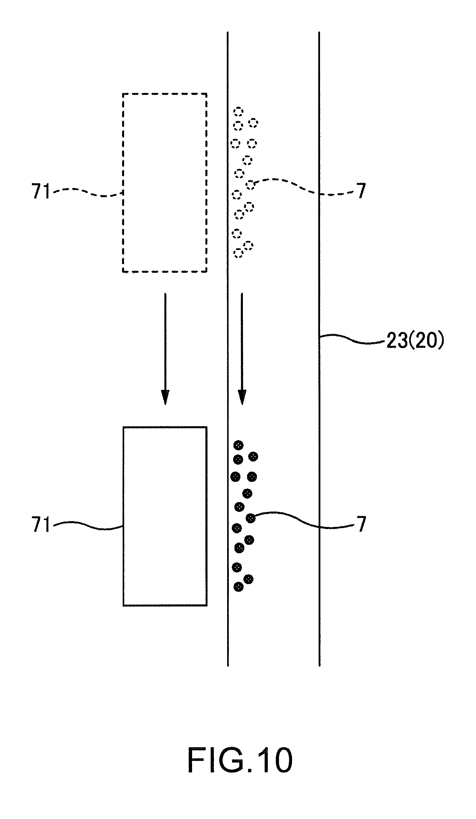

Fig. 10 is a conceptual view of the behavior of the magnetic beads 7 when the magnets 71 are moved downward. The magnetic beads 7 in the cartridge 1 are attracted to the magnets 71. Therefore, when the magnets 71 move outside the cartridge 1, the magnetic beads 7 in the cartridge 1 move with the magnets 71.

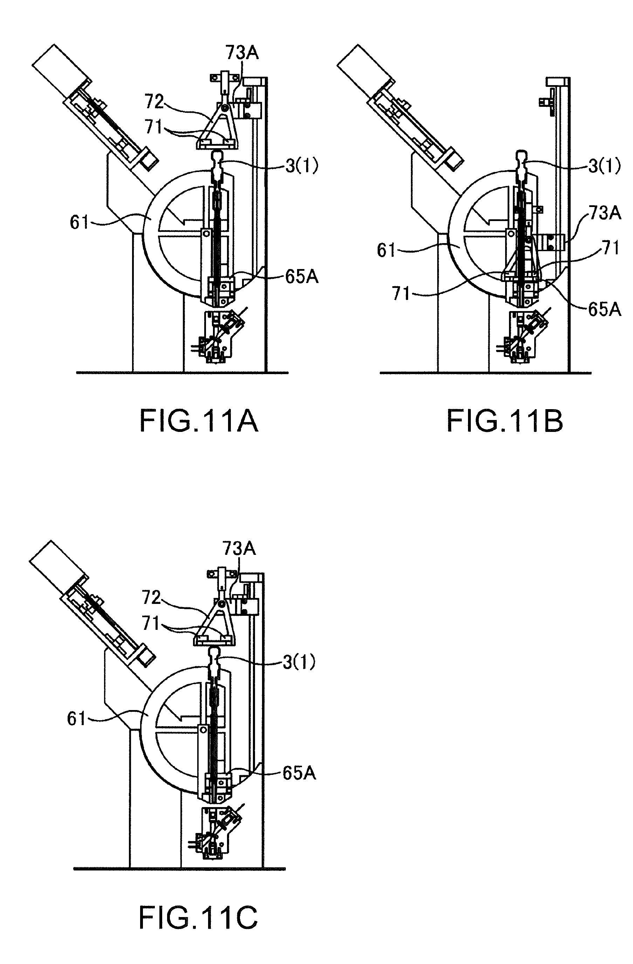

Figs. 11A to 11C are views illustrating nucleic acid elution treatment. Fig. 11A is a view illustrating a state of the PCR apparatus 50 before the nucleic acid elution treatment. Fig. 11B is a view illustrating a state of the PCR apparatus 50 when the magnets 71 are moved to the eluent plug 47. Fig. 11C is a view illustrating a state of the PCR apparatus 50 when the magnets 71 are raised.

As shown in Fig. 11A, the tank 3 is directed upward and the longitudinal direction of the cartridge 1 in the initial stat is parallel to the vertical direction. In this state, as shown in Fig. 2A, the cartridge 1 is provided with, in the following order from the top, the solution 41 (tank 3) including the magnetic beads 7, the oil 42 (plunger 10), the washing liquid 43 (upstream side of the tube 20), the first oil plug 44 (capillary 23), the washing liquid plug 45 (capillary 23), the second oil plug 46 (capillary 23), the eluent plug 47 (capillary 23), the third oil plug 48 (capillary 23), and the oil (PCR container 30).

As shown in Fig. 11A, in the initial state, the carriage 73A is positioned at the highest position (retractable position), the magnets 71 are positioned on the upper side of the cartridge 1. In this state, the controller 90 controls the lifting motor 73B to be driven to gradually move the carriage 73A downward and gradually move the magnets 71 downward. The longitudinal direction of the cartridge 1 is parallel to the vertical direction and thus the magnets 71 move along the cartridge 1.

When the magnets 71 move downward, the magnets 71 are opposite to the tank 3 and the magnetic beads 7 in the tank 3 are attracted to the magnets 71. The controller 90 controls the carriage 73A to move downward at a speed at which the magnetic beads 7 can move with the magnets 71.

When the magnets 71 move from the position opposite to the tank 3 (height of the tank 3) to the position opposite to the plunger 10 (height of the plunger 10), the magnetic beads 7 pass through the opening on the upstream side of the cylindrical portion 11 of the plunger 10 and pass though an interface between the solution 41 and the oil 42 on the upstream side of the cartridge main body 9 in the tank 3. Accordingly, the magnetic beads 7 having nucleic acids bound thereto are introduced into the cartridge main body 9. When the magnetic beads 7 pass through the interface with the oil 42 and the solution 41 is wiped off by the oil 42, and thus, the components of the solution 41 are not easily carried into the oil 42. Thus, it is possible to prevent the components of the solution 41 from being mixed with the washing liquid and the eluent 47.

When the magnets 71 move downward while being opposite to the plunger 10, the magnetic beads 7 pass through the inside of the cylindrical portion 11 and the both sides of the ribs 13 and come out from the opening on the downstream side of the cylindrical portion 11 and then are introduced to the upper syringe 21 of the tube 20. Meanwhile, the magnetic beads 7 pass though an interface between the oil 42 and the washing liquid 43 in the plunger 10. When the magnetic beads 7 are introduced into the washing liquid 43, the nucleic acids bound to the magnetic beads 7 are washed with the washing liquid 43.

In this stage, the rod-like portion 12 of the plunger 10 is not inserted into the lower syringe 22 of the tube 20, and thus, when the magnets 71 move from the position opposite to the upper syringe 21 (height of the upper syringe 21) to the position opposite to the capillary 23 (height of the capillary 23), the magnetic beads 7 move from the upper syringe 21 to the lower syringe 22 and move from the lower syringe 22 to the capillary 23. The first oil plug 44 is disposed on the upstream side of the capillary 23, and when the magnetic beads 7 move from the lower syringe 22 to the capillary 23, the magnetic beads 7 pass through the interface between the washing liquid 43 and the oil 42. At this time, the washing liquid 43 is wiped off by the oil and thus the components of the washing liquid 43 are not easily carried into the oil. Thus, it is possible to prevent the components of the washing liquid 43 from being mixed with the washing liquid plug 45 and the eluent plug 47.

When the magnets 71 move from the position opposite to the first oil plug 44 (height of the first oil plug 44) to the position opposite to the washing liquid plug 45 (height of the washing liquid plug 45), the magnetic beads 7 pass though an interface between the oil and the washing liquid. When the magnetic beads 7 are introduced into the washing liquid plug 45, the nucleic acids bound to the magnetic beads 7 are washed with the washing liquid.

When the magnets 71 move from the position opposite to the washing liquid plug 45 (height of the washing liquid plug 45) to the position opposite to the second oil plug 46 (height of the second oil plug 46), the magnetic beads 7 pass though an interface between the oil and the washing liquid. At this time, the washing liquid is wiped off by the oil and thus the components of the washing liquid are not easily carried into the oil. Thus, it is possible to prevent the components of the washing liquid from being mixed with the eluent plug 47.

When the magnets 71 move from the position opposite to the second oil plug 46 (height of the second oil plug 46) to the position opposite to the eluent plug 47 (height of the eluent plug 47), the magnetic beads 7 pass though an interface between the oil and the eluent 47.

The controller 90 controls the eluting heater 65A to heat the eluent plug 47 to about 50°C before the magnetic beads 7 are introduced into the eluent plug 47. In addition, when the eluent 47 is heated before the magnetic beads 7 are introduced, it is possible to shorten the time from when the magnetic beads 7 are introduced into the eluent 47 to when the elution of the nucleic acids ends.

As shown in Fig. 11B, after the magnets 71 move to the position opposite to the eluent plug 47 (height of the eluent plug 47), the controller 90 controls the lifting motor 73B to stop and controls the magnet 71 to stop moving in the up and down direction to perform treatment at 50°C for 30 seconds. Then, the nucleic acids bound to the magnetic beads 7 are released into the liquid of the eluent plug 47 and a reverse transcription reaction proceeds. By heating the eluent 47, the elution of the nucleic acids from the magnetic beads 7 and the reverse transcription reaction are accelerated.

After the nucleic acids are eluted with the eluent plug 47, the controller 90 controls the lifting motor 73B to be driven in the direction opposite to the rotation direction and the carriage 73A to move upward gradually to move the magnets 71 upward gradually. The controller 90 controls the carriage 73A to move upward at a speed at which the magnetic beads 7 can move with the magnets 71.

When the magnets 71 move upward gradually from the state shown in Fig. 11B, the magnetic beads 7 move from the eluent plug 47 to the second oil plug 46 and thus the magnetic beads 7 are removed from the eluent plug 47.

When the magnets 71 gradually move to the position opposite to the upper syringe 21, the magnetic beads 7 also move to the upper syringe 21 and the magnetic beads 7 are disposed on the upper side of the lower syringe 22. If the magnetic beads 7 are moved to the position, the magnetic beads 7 are not introduced into the PCR container 30 at the time when the plunger 10 is pressed. Therefore, the controller 90 may control the carriage 73A to move upward at a speed at which the magnetic beads 7 cannot follow the movement of the magnets 71 while the state is changed from the state shown in Fig. 11B to the state shown in Fig. 11C. As long as the magnetic beads 7 are not introduced into the PCR container 30 when the plunger 10 is pressed, the moving speed of the carriage 73A may be increased at an earlier state.

The information on the moving speed of the magnets 71 is stored in the storage device of the controller 90 and the controller 90 performs the above-described operation (operation of moving the magnets 71 up and down) according to the information.

### • Oscillation of Magnets 71

While the magnets 71 are moved in the up and down direction, the controller 90 may control the oscillating motor 75A to be driven to oscillate the pair of magnets 71 with interposing of the cartridge 1 therebetween.

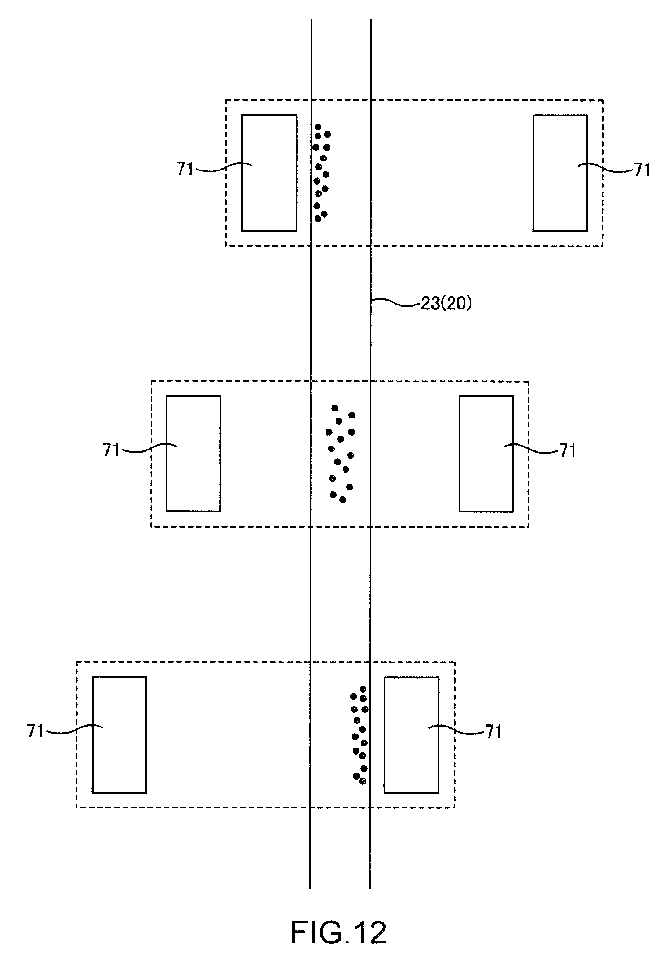

Fig. 12 is a conceptual view of behavior of the magnetic beads 7 when the magnets 71 are oscillated.

While the magnets 71 are moved in the up and down direction, the tube 20 is interposed between the pair of magnets 71 from the front and rear direction. The pair of magnets 71 are held by the arm 72 and thus the distance between the pair of magnets 71 in the front and rear direction is almost constant. Therefore, when one of the pair of magnets 71 approaches the tube 20, the other is separated from the tube 20.

The magnetic beads 7 are attracted to a closer one of the magnets 71, and thus, when one magnet 71 approaches the tube 20, the magnetic beads 7 are attracted to the one magnet 71. When the magnets 71 are separated from the tube 20 and the opposite magnet 71 approaches to the tube 20, the magnetic beads 7 are attracted to the opposite magnet 71. Thus, the magnetic beads 7 move in the front and rear direction. When the pair of magnets 71 are oscillated in the front and rear direction, the magnetic beads 7 reciprocate in the front and rear direction.

When the magnetic beads 7 reciprocate in the front and rear direction, the magnetic beads 7 are easily brought into contact with the liquid. Particularly, the liquid in the capillary 23 has little fluidity, and thus, when the liquid in the capillary 23 is brought into contact with the magnetic beads 7 as far as possible, the reciprocating of the magnetic beads 7 in the front and rear direction is effective.

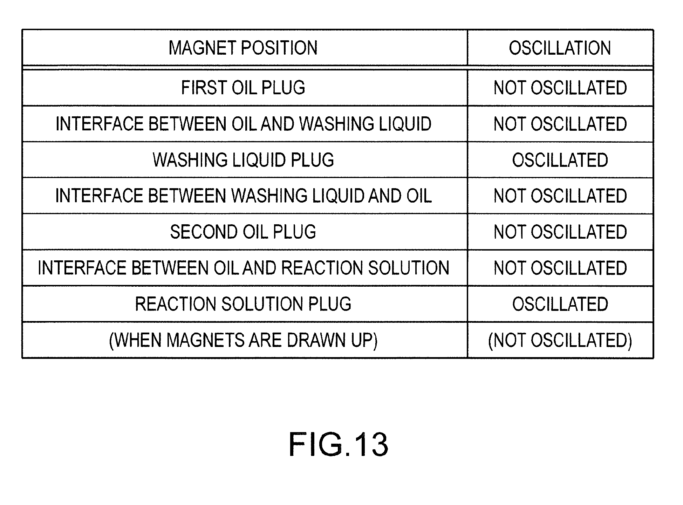

Fig. 13 is a table showing whether or not the magnets 71 are oscillated.

When the magnetic beads 7 move in the oil plug (first oil plug 44 or the second oil plug 46) in the up and down direction, the controller 90 controls the oscillating motor to stop not to oscillate the magnets 71. At this time, the controller 90 controls the magnet 71 to move downward in a state in which one of the pair of magnets 71 approaches to the tube 20. This is because the magnetic beads 7 easily follow the movement of the magnets 71 compared to a case in which a distance between each magnet 71 and the tube 20 is uniform.

When the magnetic beads 7 move downward in the washing liquid plug 45, the controller 90 controls the oscillating motor to be driven to oscillate the magnets 71 in the front and rear direction. Thus, the magnetic beads 7 move downward in the washing liquid plug 45 while being oscillated in the front and rear direction, and thus, the washing effect of the magnetic beads 7 can be enhanced. Since the washing effect is enhanced, the amount of the washing liquid plug 45 can be controlled and thus the size of the cartridge 1 can be reduced.

When the magnetic beads 7 pass through the interface between the washing liquid and the oil (second oil plug 46), the controller 90 controls the oscillating motor to stop not to oscillate the magnets 71. Accordingly, the magnetic beads 7 are not oscillated and pass through the interface, and thus, the components of the washing liquid are not easily carried into the oil. The controller 90 controls the magnets 71 to move downward in a state in which one of the pair of magnets 71 approaches to the tube 20. Thus, since the magnetic beads 7 are attracted to the close magnet 71 and aggregated and the washing liquid adhering to the magnetic beads 7 is squeezed, the components of the washing liquid is not easily carried into the oil.

When the magnetic beads 7 are in the eluent plug 47, the controller 90 controls the oscillating motor to be driven to oscillate the magnet 71 in the front and rear direction. Thus, since the magnetic beads 7 are oscillated in the eluent plug 47 in the front and rear direction, the elution effect of the nucleic acids bound to the magnetic beads 7 can be enhanced. Further, since the elution effect is enhanced, it is possible to shorten the time from when the magnetic beads 7 are introduced into the eluent 47 to when the elution of the nucleic acids ends.

When the magnetic beads 7 are attracted to the magnets by moving the magnets 71 upward after the nucleic acids are eluted in the eluent plug 47, the controller 90 controls the oscillating motor to stop not to oscillate the magnets 71. At this time, the controller 90 controls the magnets 71 to move downward in a state in which one of the pair of magnets 71 approaches to the tube 20. Thus, the magnetic beads 7 easily follow the movement of the magnets 71 and the moving speed of the magnets 71 can be increased.

The information on the position of each plug in the capillary 23 and oscillation information as shown in Fig. 13 are stored in the storage device of the controller 90, and the controller 90 performs the above-described operation (operation of oscillating the magnets 71) according to the information.

### 3. Droplet Forming Treatment

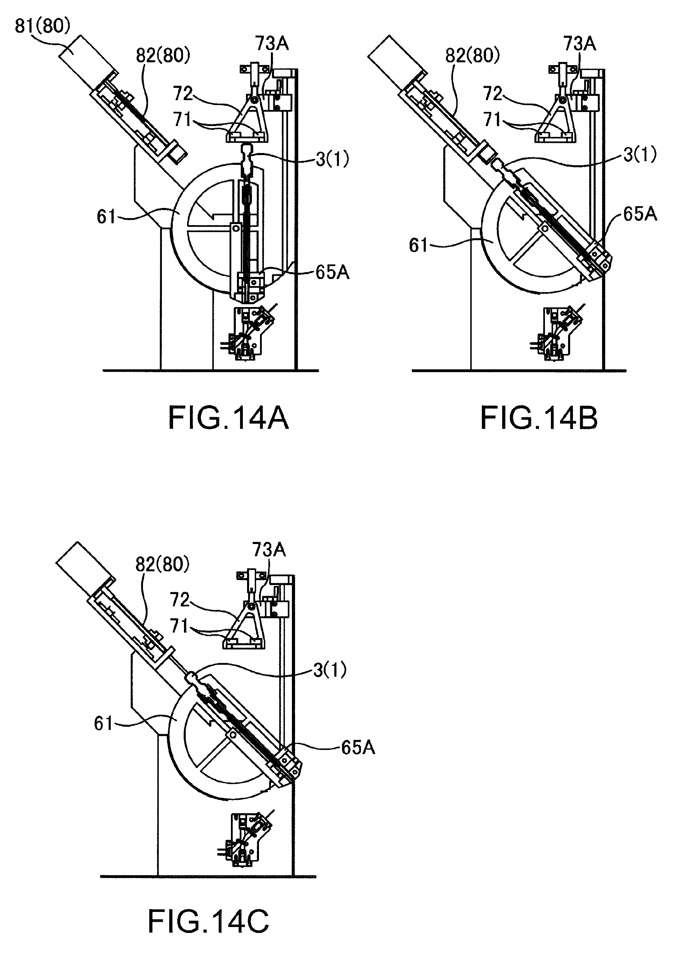

Figs. 14A to 14C are views illustrating droplet forming treatment. Fig. 14A is a view illustrating the state of the PCR apparatus 50 when the magnets 71 are drawn up. Fig. 14B is a view illustrating a state in which the rotary body 61 is rotated by 45 degrees. Fig. 14C is a view illustrating a state in which the rod 82 of the pressing mechanism 80 presses the plunger 10.

As shown in Fig. 14A, when the carriage 73A is positioned at the retractable position, the lifting mechanism 73 is not brought into contact with the cartridge 1 even when the cartridge 1 rotates. While maintaining this state, the controller 90 controls the rotary body 61 to rotate by 45 degrees.

As shown in Fig. 14B, when the rotary body 61 rotates by 45 degrees, the longitudinal direction of the cartridge 1 is parallel to the moving direction of the rod 82 of the pressing mechanism 80. The controller 90 controls the plunger motor 81 to be driven to move the rod 82. When the rod 82 further moves after the rod 82 is being brought into contact with the mount 11A of the plunger 10 of the cartridge 1, the plunger 10 is pressed into the tube 20. The controller 90 controls the rod 82 to move to be in the state shown in Fig. 14C and to press the plunger 10 until the mount 11A of the plunger 10 is brought into contact with the upper edge of the tube 20.

When the plunger 10 is pressed into the tube 20, the seal 12A of the rod-like portion 12 of the plunger 10 is fitted to the lower syringe 22 of the tube 20 (refer to Fig. 2B). Then, when the plunger 10 is further pressed, the seal 12A slides in the lower syringe 22. Thus, the liquid on the downstream side of the tube 20 (such as the third oil plug 48 or the eluent plug 47) is pushed out to the flow path forming portion 35 of the PCR container 30 by an amount equivalent to the volume in which the seal 12A slides in the lower syringe 22.

First, the third oil plug 48 in the tube 20 flows into the flow path forming portion 35. Since the flow path forming portion 35 is filled with the oil, the oil in an amount of inflow flows into the oil receiving space 32A from the flow path forming portion 35, the oil interface of the oil receiving portion 32A rises. At this time, the pressure of the liquid in the flow path forming portion 35 is higher than the outside pressure (pressure of the oil receiving portion 32A due to pressure loss in the lower sealing portion 34B. After the third oil plug 48 is pushed out from the tube 20, the eluent plug 47 flows into the flow path forming portion 35 from the tube 20. Since the inner diameter of the flow path forming portion 35 is larger than the inner diameter of the capillary 23, the plug-shaped eluent 47 in the tube 20 is formed into droplets in the oil in the flow path forming portion 35.

The volume in which the seal 12A slides in the lower syringe 22 (amount in which the liquid in the tube 20 is pushed out from the downstream side) is larger than a total amount of the eluent plug 47 and the third oil plug 48 in the tube 20, and thus, after the eluent plug 47 is pushed out from the tube 20, some of the second oil plug 46 is also pushed out to the flow path forming portion 35. Thus, the eluent 47 does not remain in the tube 20 and a total liquid amount of the eluent plug 47 is formed into droplets. In addition, when some of the second oil plug 46 is pushed out from the downstream side of the tube 20, the eluent 47 in the form of droplets is easily separated from the tube 20 (the eluent 47 in the form of droplets are not easily adsorbed onto the opening of the capillary 23) .

Since the inner diameter of the terminal end of the capillary 23 (the diameter of the opening of the capillary 23) is designed to have a relatively small size, the eluent 47 having the form of droplets in the PCR container 30 is not easily adsorbed onto the opening of the capillary 23. Further, the specific gravity of the eluent 47 is greater than the specific gravity of the oil in the PCR container 30. Thus, the eluent 47 in the form of droplets is separated from the terminal end of the capillary 23 and is precipitated toward the bottom 35A using flow path forming portion 35 as a flow path. Then, the precipitated eluent reaches the bottom 35A of the PCR container 30 and the precipitation ends. The eluent is brought into contact with the freeze-dried nucleic acid amplification reaction reagent to dissolve the nucleic acid amplification reaction reagent. However, since the flow path of the flow path forming portion 35 is inclined at 45 degrees in this stage, the eluent 47 in the form of droplets easily adheres to the inner wall of the flow path forming portion 35. Therefore, it is necessary to return the flow path of the flow path forming portion 35 to the vertical direction.

After the eluent 47 in the form of droplets is formed (after the plunger 10 is pressed), the controller 90 controls the plunger motor 81 to be driven in the direction opposite to the rotation direction to return the rod 82 to the original position. In this state, even when the cartridge 1 rotates, the rod 82 of the pressing mechanism 80 is not brought into contact with the cartridge 1. While maintaining this state, the controller 90 controls the rotary body 61 to return the reference position. When the rotary body 61 is returned to the reference position, the flow path of the flow path forming portion 35 is directed to the vertical direction and thus the eluent 47 in the form of droplets does not easily adhere to the inner wall of the flow path forming portion 35.

### 4. Thermal Cycling Treatment

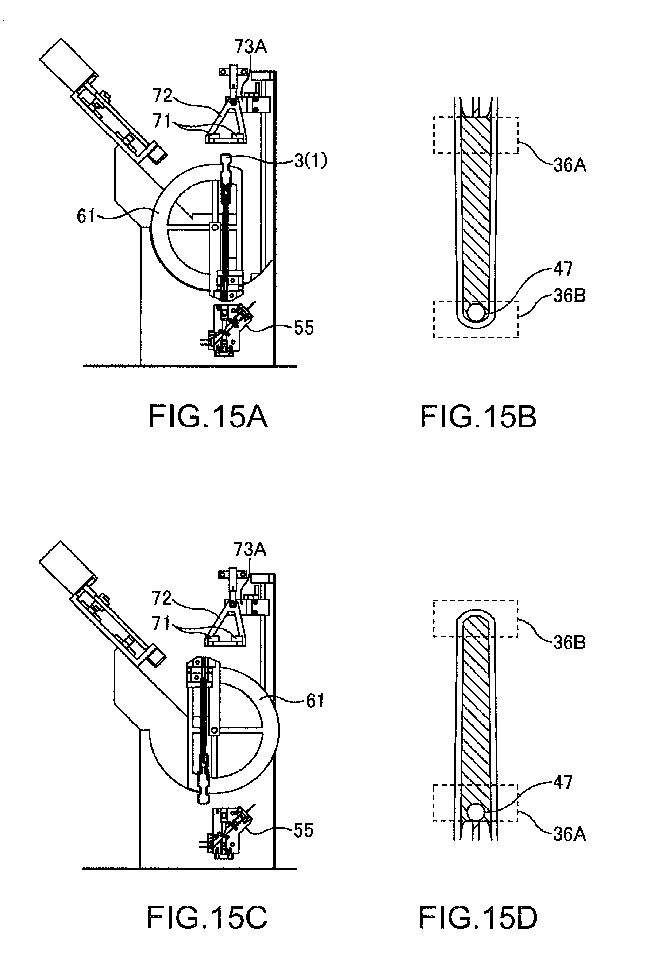

Figs. 15A to 15D are views illustrating thermal cycling treatment. Figs. 15A and 15B are views illustrating a state in which the eluent 47 is subjected to low temperature-side temperature treatment. Figs. 15C and 15D are views illustrating a state in which the eluent 47 is subjected to high temperature-side temperature treatment. The state of the PCR apparatus 50 is shown on the left side in each drawing, and the state of the inside of the flow path forming portion 35 of the PCR container 30 is shown on the right side in each drawing.

When the cartridge 1 is fixed to the mounting portion 62 in a normal position, the upstream side of the flow path forming portion 35 of the PCR container 30 (high temperature region 36A) is opposite to the high temperature-side heater 65B and the downstream side of the flow path forming portion 35 of the PCR container 30 (low temperature region 36B) is opposite to the low temperature-side heater 65C. During the thermal cycling treatment, the controller 90 controls the high temperature-side heater 65B that is provided in the rotary body 61 to heat the liquid in the high temperature region 36A on the upstream side of the flow path forming portion 35 of the PCR container 30 to about 90°C to 100°C. In addition, the controller 90 controls the low temperature-side heater 65C that is provided in the rotary body 61 to heat the liquid in the low temperature region 36B on the downstream side of the flow path forming portion 35 to about 50°C to 75°C. Thus, during the thermal cycling treatment, a temperature gradient is generated in the liquid in the flow path forming portion 35 of the PCR container 30. The mounting portion 62 and the heaters are provided in the rotary body 61, and thus, even when the rotary body 61 rotates, the positional relationship between the cartridge 1 and the heaters is maintained as it is. When RT-PCR is performed in the PCR container 30, the eluent 47 which dissolved the nucleic acid amplification reaction reagent before the thermal cycling treatment may be treated by the low temperature-side heater 65C at about 42°C to 55°C so that a reverse transcription reaction is performed.

During the thermal cycling treatment, the liquid in the PCR container 30 is heated. If bubbles are generated by heating the liquid in the PCR container 30, there is a concern that temperature unevenness may occur in the liquid in the flow path forming portion 35 or the movement (precipitation) of the eluent 47 in the form of droplets in the flow path forming portion 35 may be inhibited. However, the pressure of the liquid in the flow path forming portion 35 is higher than the outside pressure due to pressure loss in the lower sealing portion 34B in the embodiment and thus bubbles are not easily generated in the liquid in the PCR container 30.

As shown in Figs. 15A and 15B, when the rotary body 61 is positioned at the reference position, the low temperature-side heater 65C is positioned on the lower side of the high temperature-side heater 65B and the bottom 35A of the PCR container 30 of the cartridge 1 is directed downward. Since the specific gravity of the eluent 47 in the form of droplets is greater that the specific gravity of the oil, the eluent 47 in the form of droplets is precipitated in the flow path forming portion 35. When the eluent 47 in the form of droplets is precipitated in the flow path forming portion 35, the precipitated eluent reaches the bottom 35A of the PCR container 30. The precipitation ends and the eluent stays in the low temperature region 36B. Accordingly, the eluent 47 in the form of droplets moves to the low temperature region 36B. The controller 90 controls the PCR container 30 of the cartridge 1 to be held in the state shown in Fig. 15B for a predetermined period of time, and the eluent 47 in the form of droplets is heated in the low temperature region 36B at about 50°C to 75°C (low temperature-side temperature treatment is performed). In the meantime, the elongation reaction of the polymerase reaction is caused.

When the controller 90 drives the rotating motor 66 from the state shown in Fig. 15B to rotate the rotary body 61 by 180 degrees, the state of the apparatus is changed to the state shown in Figs. 15C and 15D. When the rotary body 61 rotates from the reference position by 180 degrees, the cartridge 1 is inverted vertically and the high temperature-side heater 65B and the low temperature-side heater 65C are also inverted vertically. That is, the high temperature-side heater 65B is positioned on the lower side of the low temperature-side heater 65C and the bottom 35A of the PCR container 30 of the cartridge 1 is directed upward. When the eluent 47 in the form of droplets is precipitated in the flow path forming portion 35, the precipitated eluent reaches the terminal end of the tube 20 (terminal end of the capillary 23). The precipitation ends and the eluent stays in the high temperature region 36A. Thus, the eluent 47 in the form of droplets moves to the high temperature region 36A. The controller 90 controls the PCR container 30 of the cartridge 1 to be held in the state shown in Fig. 15D for a predetermined period of time, and the eluent 47 in the form of droplets is heated to about 90°C to 100°C in the high temperature region 36A (high temperature-side temperature treatment is performed). In the meantime, the elongation reaction of the polymerase reaction is caused.

When the controller 90 drives the rotating motor 66 from the state shown in Fig. 15D to rotate the rotary body 61 by -180 degrees, the PCR container 30 of the cartridge 1 is returned to the state shown in Fig. 15B. When the eluent 47 in the form of droplets is precipitated in the flow path forming portion 35 in this state, the eluent 47 in the form of droplets moves to the low temperature region 36B and is reheated to about 50°C to 75°C in the low temperature region 36B (low temperature-side temperature treatment is performed). Since the inner diameter of the terminal end of the capillary 23 (the diameter of the opening of the capillary 23) is designed to have a relatively small size, the eluent 47 is not easily adsorbed onto the opening of the capillary 23. Thus, when the PCR container 30 rotates by -180 degrees from the state shown in Fig. 15D, the eluent 47 in the form of droplets is not adsorbed onto the opening of the capillary 23 and is separated from the tube 20 and precipitated toward the bottom 35A of the PCR container 30.

The controller 90 drives the rotating motor 66 to repeatedly perform an operation of changing the rotational position of the rotary body 61 to the state shown in Fig. 15A and the state shown in Fig. 15B in the prescribed number of cycles. Thus, the PCR apparatus 50 can perform thermal cycling treatment of PCR on the eluent 47.

The thermal cycling information on the temperature of the high temperature-side heater 65B, the temperature of the low temperature-side heater 65C, the time for which the PCR container 30 is held in the state shown in Fig. 15B, the time for which the PCR container is held in the state shown in Fig. 15D, and the number of cycles (the number of repetitions of the state shown in Fig. 15B and the state shown in Fig. 15D) is stored in the storage device of the controller 90, and the controller 90 performs the above-described operation according to the thermal cycling information.

### 5. Fluorescence Measurement in Real Time PCR

As shown in Fig. 15A, when the rotary body 61 is positioned in the reference position, the fluorometer 55 is opposite to the bottom 35A of the PCR container 30 of the cartridge 1. Therefore, at the time of the fluorescence measurement of the eluent 47, the controller 90 controls the fluorometer 55 to measure the intensity of the fluorescence from the eluent 47 on the bottom 35A of the PCR container 30 from the lower opening of the insertion hole 64A of the low temperature-side heater 65C in a state in which the rotary body 61 is returned to the reference position.

Immediately after the rotary body 61 rotates by 180 degrees and is returned to the reference position, the eluent 47 in the form of droplets is precipitated in the flow path forming portion 35 of the PCR container 30 and the eluent 47 in the form of droplets does not reach the bottom 35A of the PCR container 30 in some cases. Therefore, it is preferable that the controller 90 perform fluorescence intensity measurement after a predetermined period of time has elapsed from when the rotational position of the rotary body 61 is changed to the state shown in Fig. 15A (immediately before the rotary body 61 is rotated from the state shown in Fig. 15A). Alternatively, the controller 90 may control the fluorometer 55 to measure the intensity of the fluorescence for a predetermined period of time after the rotary body 61 is returned to the reference position so as to store the time history of the intensity of the fluorescence.

### Other Embodiments

The foregoing embodiments are described to facilitate understanding of the invention and are not intended to limit the interpretation of the invention. Various modifications and improvements can be made to the invention without departing from the spirit of the invention, and needless to say, the equivalents are included within the scope of the invention.

### Regarding PCR Apparatus

The aforementioned PCR apparatus 50 inverts the PCR container 30 vertically by changing the posture of the cartridge 1 in a predetermined number of cycles as thermal cycling treatment. However, the number of times of changing the posture of the cartridge 1 is not limited to a multiple of times and may be one time.

In the aforementioned PCR apparatus, the rotation axis of the rotary body is positioned at a position close to the tube than to the PCR container. However, as long as the droplets can move in the PCR container when the rotary body is rotated and the posture of the cartridge is changed, the rotation axis of the rotary body is not limited thereto.

In the aforementioned PCR apparatus, the fixing portion 63 in which a notch is formed and the insertion hole 64A are used as the mounting portion of the cartridge. However, as long as the cartridge can be mounted to the rotary body, the configuration is not limited thereto. In addition, the mounting portion may have a configuration in which the cartridge is fixed to the rotary body by fixing only a portion of the cartridge on the side close to the tube or a configuration in which the cartridge is fixed to the rotary body by fixing only a portion of the cartridge on the side close to the PCR container. However, it is necessary that the mounting portion have a configuration in which the cartridge is stably fixed to the rotary body even when the rotary body rotates and the posture of the cartridge is changed.

The aforementioned PCR apparatus 50 includes the high temperature-side heater 65B and the low temperature-side heater 65C as heaters for PCR. However, as long as a temperature gradient is generated in the PCR container which is a container for nucleic acid amplification reaction, the configuration is not limited thereto. For example, the heaters may be provided only on the high temperature side. Alternatively, a heater may be provided on the high temperature side and a cooler may be provided on the low temperature side.

Further, in the aforementioned PCR apparatus 50, the rotary body is provided with the heaters for PCR (high temperature-side heater 65B and low temperature-side heater 65C). However, as long as a temperature gradient is generated in the PCR container which is a container for nucleic acid amplification reaction, the heaters for PCR may be provided outside the rotary body. For example, the PCR apparatus may include a first heater for PCR which is opposite to the PCR container when the rotary body 61 is positioned at the reference position as shown in Fig. 15A and a second heater for PCR which is opposite to the PCR container when the rotary body is rotated by 180 degrees as shown in Fig. 15C outside the rotary body. Even with such a configuration, a temperature gradient can be generated in the PCR container which is a container for nucleic acid amplification reaction. However, when the heaters for PCR are provided in the rotary body, irrespective of the rotational position of the rotary body, the positional relationship between the PCR container of the cartridge and the heaters is maintained, and thus, this configuration is preferable.

The PCR apparatus 50 may include only the heaters for PCR (high temperature-side heater 65B and low temperature-side heater 65C) without the eluting heater 65A. However, when the PCR apparatus 50 includes the eluting heater 65A, the release of the nucleic acids from the magnetic beads is promoted and thus this configuration is preferable.

The PCR apparatus 50 may include the magnet moving mechanism which moves the magnets along the tube. In this case, for example, an operator may hold the magnets and move the magnets along the tube. However, there is a concern that the moving speed of the magnetic beads which are nucleic acid-binding solid-phase carriers may be different according to an operator and thus it is preferable that the PCR apparatus 50 include the magnet moving mechanism.

In addition, the magnet moving mechanism 70 of the PCR apparatus 50 may not include the oscillation mechanism 75. In this case, although the magnets cannot be oscillated, the magnets can be moved along the tube, and thus, the magnetic beads bound to the nucleic acids can be moved to the plug including the eluent.

The PCR apparatus 50 may not include the pressing mechanism 80 and may include an alternative pushing unit. In this case, for example, the plunger or the tank may be used as a pushing unit. That is, an operator may press the plunger of the cartridge. When the plunger is not provided in the cartridge, the tank made of a deformable material as described above is deformed by the operator and thus the inside of the tank is pressurized and the liquid is pushed out from the tube to the PCR container.

### Third Embodiment

In a third embodiment of the invention, a freeze-dried nucleic acid amplification reaction reagent 300 in the aforementioned second embodiment is not disposed in the container for nucleic acid amplification reaction but is disposed in a third oil plug 311 provided on the downstream side of an eluent plug 47 of a tube 301.

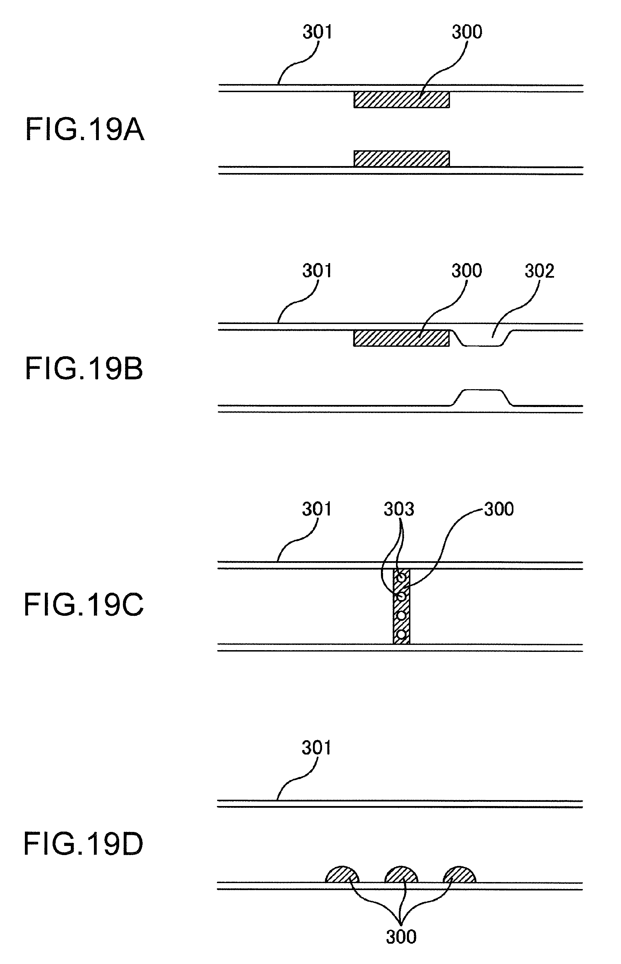

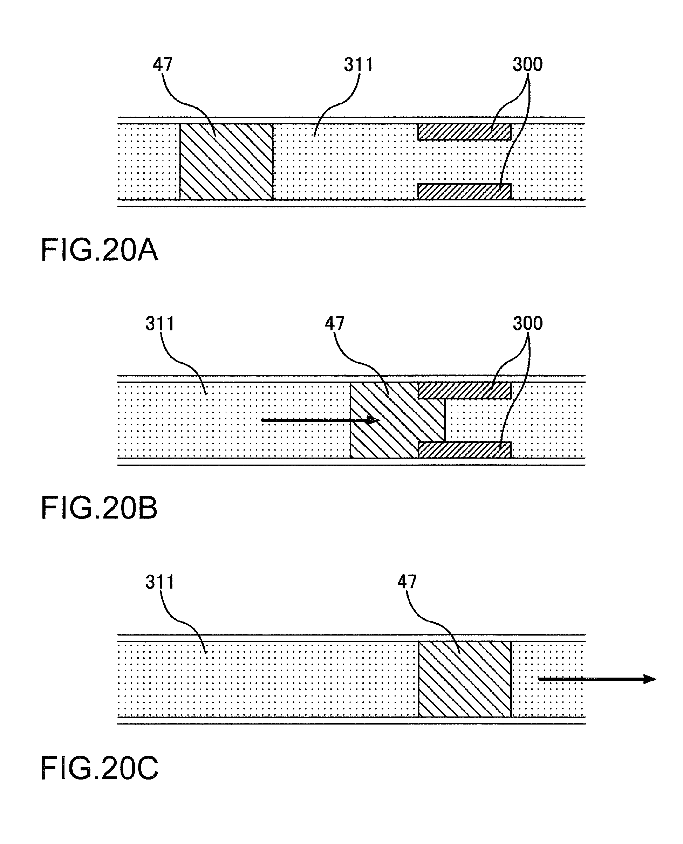

The shape of the nucleic acid amplification reaction reagent 300 is not particularly limited but when a plug shape in which the tube 301 is capped is adopted, the nucleic acid amplification reaction reagent 300 moves with the oil plug by the pressure to push out the eluent, and dissolution of the nucleic acid amplification reaction reagent in the eluent 47 is interrupted. When there is a space through which the oil can freely flow in the region of the tube 301 in which the nucleic acid amplification reaction reagent 300 is held, specifically, the nucleic acid amplification reaction reagent is preferably disposed in a hollow shape along the inner wall of the tube 301 (Fig. 19A). That is, when the nucleic acid amplification reaction reagent 300 is disposed in the hollow shape, as shown in the schematic views of Figs. 20A to 20C, an oil 311 passes though the center portion of the tube 301 while avoiding the nucleic acid amplification reaction reagent 300 by the operation of pushing out the liquid to the container for nucleic acid amplification reaction by the plunger and the eluent 47 which reaches the nucleic acid amplification reaction reagent 300 dissolves the nucleic acid amplification reaction reagent 300. Then, the eluent 47 which dissolved the nucleic acid amplification reaction reagent 300 is pushed out to the container for nucleic acid amplification reaction from the tube. The plunger may stop for a predetermined period of time when the eluent 47 is brought into contact with the nucleic acid amplification reaction reagent 300. Accordingly, the nucleic acid amplification reaction reagent 300 can be immersed in the eluent 47 until the nucleic acid amplification reaction reagent 300 is dissolved in the eluent 47. In addition, when the eluent 47 is brought into contact with the nucleic acid amplification reaction reagent 300, a pulling operation may be preformed. Thus, the nucleic acid amplification reaction reagent 300 is easily dissolved in the eluent 47 by pressing or pulling the plunger. Further, when the eluent 47 is brought into contact with the nucleic acid amplification reaction reagent 300, a heating unit which heats the eluent 47 may be provided. Thus, the nucleic acid amplification reaction reagent 300 is easily dissolved in the eluent 47. The heating unit may be, for example, the high temperature-side heater 65B or the low temperature-side heater 65C. Such an operation of the plunger may be controlled by a control device such as a computer. Further, in order to prevent the nucleic acid amplification reaction reagent 300 from moving, a thick portion 302 is provided in the tube 301 on the downstream side of the nucleic acid amplification reaction reagent 300 and the inner diameter of the tube 301 may be narrowed (Fig. 19B). When the nucleic acid amplification reaction reagent is formed into a plug shape, bubbles 303 may be generated in the freeze-dried nucleic acid amplification reaction reagent 300 so as to avoid application of a load to the solution resulting from expansion (Fig. 19C) . In addition, plural nucleic acid amplification reaction reagents 300 may be disposed on the wall surface (Fig. 19D).

### Examples

### Example 1

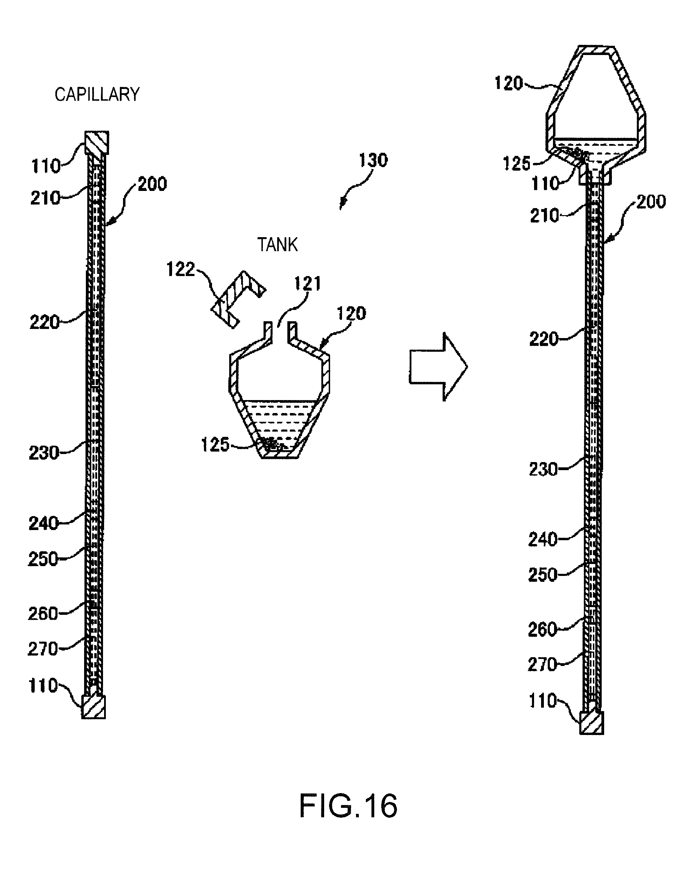

In Example, as shown in Fig. 16, a configuration in which a first plug 210 to a seventh plug 270 are internally provided in a tube 200 in the aforementioned kit for nucleic acid extraction was used.

First, in a polyethylene container 130 having a volume of 3 mL, 375 µL of a solution and 1 µL of a dispersion of magnetic beads were placed. As the solution, an aqueous solution of 76 mass% guanidine hydrochloride, 1.7 mass% ethylenediaminetetraacetic acid disodium salt dihydrate, and 10 mass% polyoxyethylene sorbitan monolaurate (MagExtractor-Genome, NPK-1, manufactured by Toyobo Co., Ltd.) was used. Further, as an undiluted solution of magnetic beads, a solution containing 50 vol% magnetic silica particles and 20 mass% lithium chloride was used.

50 µL of blood collected from a human was placed into the container 130 through an opening 121 using a pipette, the container 130 was capped with a cap 122 and shaken for 30 seconds by hand for stirring. Then, the cap 122 of the container 130 was removed and the container was connected to the tube 200. Stoppers 110 were provided at both ends of the tube 200. The stopper 110 on the side close to the first plug 210 was removed and the container 130 was connected to the tube 200.

As the first plug 210, the third plug 230, the seventh plug 270, and the fifth plug 250, silicon oil was used. As a first washing liquid of the second plug 220, an aqueous solution of 76 mass% guanidine hydrochloride was used. Further, as a second washing liquid of the fourth plug 240, a Tris-HCl (pH 8.0, solute concentration 5 mM) was used. As an eluent of the sixth plug 260, sterilized water was used.

Magnetic beads 125 in the container 130 were introduced into the tube 200 by moving permanent magnets 410. Then, the magnetic beads 125 were moved to the sixth plug 260. The time for which the magnetic beads 125 were present in each plug in the tube 200 was as follows: the first, third, and seventh plugs: 3 seconds, respectively; the second plug: 20 seconds; the fourth plug: 20 seconds; and the sixth plug: 30 seconds. In the second plug 220 and the fourth plug 240, an operation of shaking the magnetic beads or the like was performed. In addition, the volumes of the second plug 220, the fourth plug 240, and the sixth plug 260 were 25 µL, 25 µL, and 1 µL, respectively.

Next, the stopper 110 on the side close to the seventh plug of the tube was removed and the container 120 was deformed by hand to discharge the seventh plug 270 and the sixth plug 260 into a PCR reaction container. This operation was performed after the magnetic beads were moved by the permanent magnets and retracted to the second plug 220.

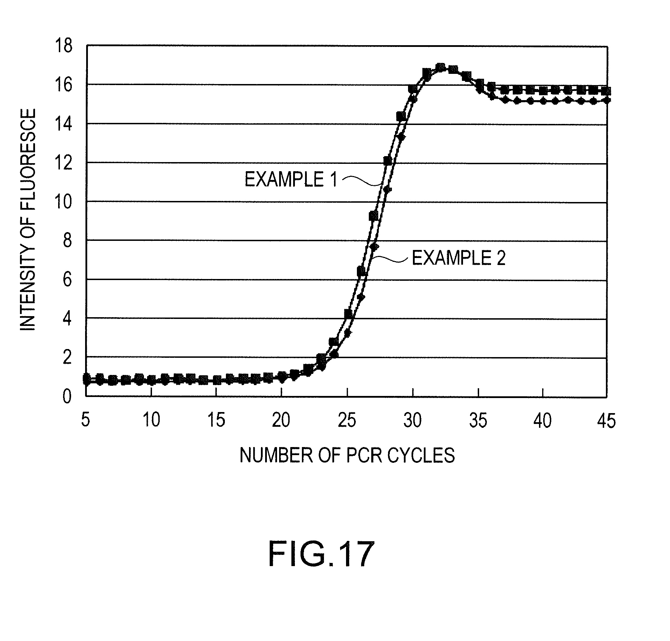

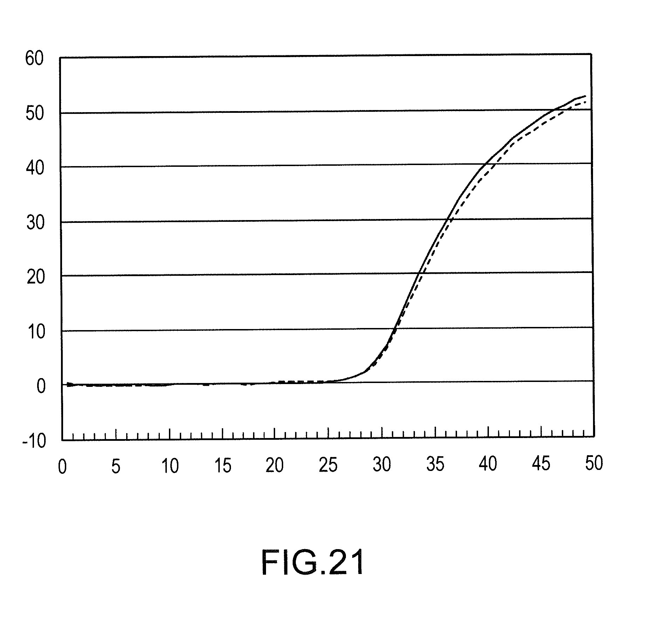

19 µL of a PCR reaction reagent was added to the extract and real time PCR was performed according to a normal procedure. The PCR reaction reagent contains 4 µL of LightCycler 480 Genotyping Master (Cat. no. 4 707 524, manufactured by Roche Diagnostics), 0.4 µL of SYBR Green I (S7563, manufactured by Life Technologies) diluted 1000 times with sterilized water, 0.06 µL of 100 µM primers for β-action detection (F/R), respectively, and 14.48 µL of sterilized water. The PCR amplification curve of Example 1 is shown in Fig. 21. The vertical axis of Fig. 17 represents intensity of fluoresce and the horizontal axis represents the number of PCR cycles.

### Example 2

In Example 2, nucleic acids were extracted by a typical nucleic acid extraction method.

First, in a polyethylene container (Eppendorf tube) having a volume of 1.5 mL, 375 µL of a solution and 20 µL of a dispersion of magnetic beads were placed. The compositions of the solution and dispersion are the same as in the above-described example.

Next, 50 µL of blood collected from a human was placed into the container through an opening using a pipette, the container was capped with a cap and stirred for 10 minutes with a Vortex mixer. A B/F separation operation was performed by using a magnetic stand or a pipette. In this state, the magnetic beads and a small amount of the solution remained in the container.

Next, 450 µL of a first washing liquid having the same configuration as in Example 1 was placed in the container and the container was capped with a cap and stirred for 5 seconds with a Vortex mixer. The first washing liquid was removed by using a magnetic stand or a pipette. This operation was performed twice. In this state, the magnetic beads and a small amount of the first washing liquid remained in the container.

Next, 450 µL of a second washing liquid having the same composition as in Example 1 was placed into the container and the container was capped with a cap and stirred for 5 seconds with a Vortex mixer. The second washing liquid was removed by using a magnetic stand or a pipette. This operation was performed twice. In this state, the magnetic beads and a small amount of the second washing liquid remained in the container.

Then, 50 µL of sterilized water (eluent) was added to the container and the container was capped with a cap and stirred for 10 minutes with a Vortex mixer. The supernatant was collected by using a magnetic stand or a pipette. The supernatant includes target nucleic acids.

1 µL of the extract therefrom was dispensed and further 19 µL of a PCR reaction reagent was added. Then, real time PCR was performed according to a normal procedure. The PCR reaction reagent contains 4 µL of LightCycler 480 Genotyping Master (Cat. no. 4 707 524, manufactured by Roche Diagnostics), 0.4 µL of SYBR Green I (S7563, manufactured by Life Technologies) diluted 1000 times with sterilized water, 0.06 µL of 100 µM primers for β-action detection (F/R), respectively, and 14.48 µL of sterilized water. The PCR amplification curve at this time is shown in Fig. 17.

### Experiment Results

From the above-described examples, it is possible to understand the following results.
(1) When compared to the time required for nucleic acid extraction treatment which is a pretreatment of PCR, the time from when a specimen was inserted into a container to when target nucleic acids was introduced into the PCR reaction container was about 2 minutes in Example 1. In Example 2, the time was about 30 minutes. Thus, the time for nucleic acid extraction in the nucleic acid extraction method of Example 1 is shorter than that in the nucleic acid extraction method of Example 2.
(2) The amount of each washing liquid used in Example 1 was about 1/18 of the amount of each washing liquid used in Example 2. Further, the amount of the eluent in Example 1 was about 1/50 of the amount of the eluent in Example 2. Accordingly, in Example 1, the amounts of the washing liquids and the eluent were very small but sufficient compared to Example 2.
(3) When the concentration of the target nucleic acids in the eluent is compared with the amounts of the solution and the eluent, it is considered that the concentration of the nucleic acids in Example 1 is ideally higher than the concentration of the nucleic acids in Example 2 by 50 times. However, in the examples, the amount of the nucleic acids included in the blood sample is large and exceeds the amount in which 1 µL of the magnetic beads can adsorb, and all the nucleic acids included in the blood sample cannot be collected. Thus, the concentration of the nucleic acids of 50 times of the concentration of the nucleic acids in Example 2 cannot be obtained in Example 1. In a case of a specimen in which the amount of the nucleic acids is small and does not exceed the amount in which 1 µL of the magnetic beads can adsorb, the concentration of the nucleic acids of 50 times of the concentration of the nucleic acids in Example 2 can be obtained in Example 1.
(4) As seen from the graph shown in Fig. 17, even in all blood samples in which the amount of the nucleic acids is large, rising of the amplification factor of the nucleic acids in Example 1 is exhibited earlier than in Example 2 by about 0.6 cycles. That is, the concentration of the nucleic acids in the PCR reaction solution used in Example 1 is higher than the concentration of the nucleic acids in the PCR reaction solution used in Example 2. That is, the concentration of the target nucleic acids in the eluent in Example 1 is higher than in Example 2.

### Example 3

In this example, the storage stability when the reagent for nucleic acid amplification was a solution was compared with the storage stability when the reagent for nucleic acid amplification was freeze-dried.

First, 10 µL of an adjusted nucleic acid amplification solution was placed in each 200 µL Eppendorf PCR tube (container for nucleic acid amplification reaction) to prepare two reagents and one was maintained in the form of a solution as it was and the other was freeze-dried. The nucleic acid amplification solution is as follows.
0.8 µM Primer F: GAC CAA TCC TGT CAC CTC TGA C
0.8 µM Primer R: AGG GCA TTT TGG ACA AAG CGT CTA
0.2 µM Probe
TaqMan probe: FAM- TGC AGT CCT CGC TCA CTG GGC ACG -TAMRA
1 x SuperScript III RT/Platimun Taq Mix
1 x PCR Master Mix

The freeze-dried reagent for nucleic acid amplification was laminated with an oil and each reagent was stored at room temperature (25°C) to investigate the RT-PCR reaction after a few days to a month. The conditions for RT-PCR are as follows.
Reverse transcription: 50°C for 60 seconds
Enzyme inactivation: 95°C for 10 seconds
Modification: 95°C for 5 seconds
Annealing and elongation: 57°C for 20 Seconds
(50 cycles)

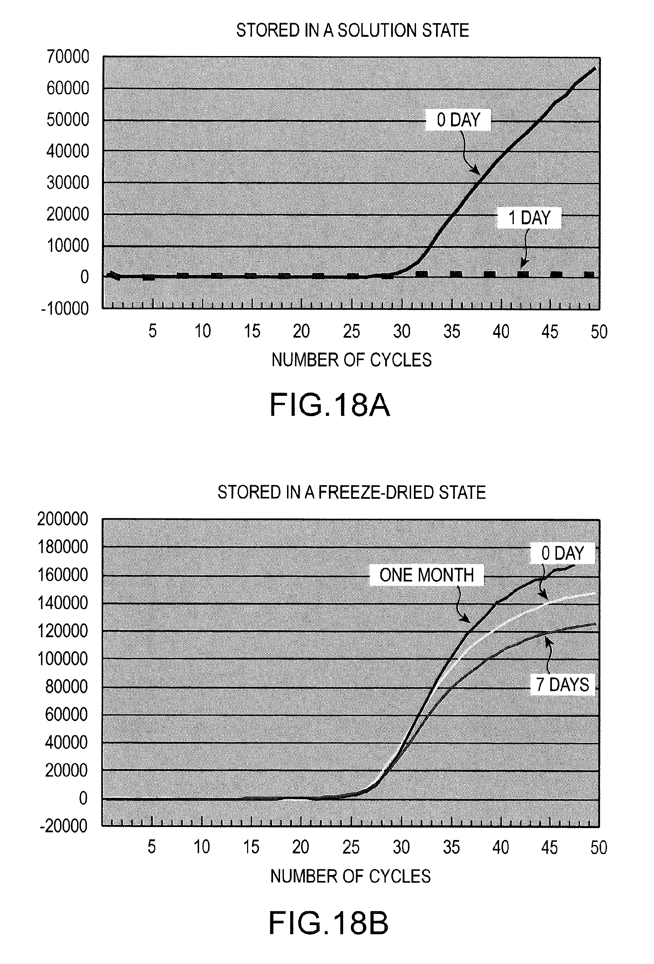

As shown in Fig. 18A, the reagent in the form of a solution was not able to be stored even for one day. On the other hand, as shown in Fig. 18B, the freeze-dried reagent was able to be stored for a month or longer.

As described above, when the reagent for nucleic acid amplification is freeze-dried, the reagent is used for a sample even after being stored at room temperature for a month or longer, and the same reaction immediately after the reagent is prepared occurs. Further, even when the freeze-dried product is covered with an oil, the reaction occurs without inhibition from the oil.

### Example 4

Using the freeze-dried reagent for nucleic acid amplification 300 prepared as shown in Fig. 19A under the same conditions as in Example 3, the reagent for nucleic acid amplification 300 was dissolved in the eluent 47 as shown in Figs. 20A to 20C and the eluent 47 obtained by pushing was used to perform RT-PCR (the dotted line in Fig. 21). As a control, the reagent for nucleic acid amplification adjusted immediately before the experiment was used to perform RT-PCR in the same manner (the solid line in Fig. 21).

As shown in Fig. 21, regarding to the amplification, both the reagents exhibited almost the same effect. In this manner, even when the freeze-dried reagent for nucleic acid amplification was fixed to the tube, the nucleic acids were able to be amplified in RT-PCR with the same efficiency as the reagent for nucleic acid amplification which was not freeze-dried.

## Claims

1. A container for nucleic acid amplification reaction comprising:
a freeze-dried nucleic acid amplification reaction reagent;
an oil; and
a container that contains the freeze-dried nucleic acid amplification reaction reagent and the oil,
wherein the freeze-dried nucleic acid amplification reaction reagent is held in the oil.

2. The container for nucleic acid amplification reaction according to Claim 1,
wherein the oil is subjected to dehydration treatment.

3. The container for nucleic acid amplification reaction according to Claim 1 or 2,
wherein the freeze-dried nucleic acid amplification reaction reagent is cake-like.

4. The container for nucleic acid amplification reaction according to any one of the preceding claims,
wherein the freeze-dried nucleic acid amplification reaction reagent is a porous substance having a hole having a diameter of 20 µm or less.

5. The container for nucleic acid amplification reaction according to any one of the preceding claims,
wherein the nucleic acid amplification reaction reagent contains a DNA polymerase and dNTP.

6. The container for nucleic acid amplification reaction according to Claim 5,
wherein the nucleic acid amplification reaction reagent contains a primer for nucleic acid amplification reaction.

7. The container for nucleic acid amplification reaction according to any one of the preceding claims,
wherein the nucleic acid amplification reaction reagent contains a reverse transcriptase.

8. The container for nucleic acid amplification reaction according to any one of the preceding claims,
wherein the nucleic acid amplification reaction reagent and the oil for nucleic acid amplification reaction are separated by a solid wax.

9. A cartridge for nucleic acid amplification reaction comprising:
a tube that is internally provided with, in the following order, in a longitudinal direction,
a first plug composed of an oil,
a second plug composed of a first washing liquid, which is phase-separated from an oil and is used for washing a fine particle having nucleic acids bound thereto,
a third plug composed of an oil,
a fourth plug composed of an eluent which is phase-separated from an oil and is used for eluting the nucleic acids from a fine particle having the nucleic acids bound thereto, and
a fifth plug composed of an oil; and
a container for nucleic acid amplification reaction according to any one of the preceding claims that is disposed to communicate with an end portion of the tube on a side on which the fifth plug is disposed and contains an oil,
wherein a freeze-dried nucleic acid amplification reaction reagent is held in the oil in the container for nucleic acid amplification reaction.

10. The cartridge for nucleic acid amplification reaction according to Claim 9,
wherein the nucleic acid amplification reaction reagent is disposed on an end portion of the container for nucleic acid amplification reaction opposite to the side communicating with the tube.

11. The cartridge for nucleic acid amplification reaction according to Claim 10,
wherein the end portion of the container for nucleic acid amplification reaction opposite to the side communicating with the tube has a tapered shape.

12. The cartridge for nucleic acid amplification reaction according to Claim 10 or 11,
wherein the specific gravity of the oil in the container for nucleic acid amplification reaction is smaller than the specific gravity of the eluent.

13. The cartridge for nucleic acid amplification reaction according to any one of Claims 9 to 12,
wherein the viscosity of the oil in the container for nucleic acid amplification reaction is 50 cs or less.

14. The cartridge for nucleic acid amplification reaction according to any one of Claims 9 to 13, further comprising
a pushing unit which is mounted on an opening portion of the tube on a side on which the first plug is disposed to push out the eluent from the end portion of the tube on the side on which the fifth plug is disposed to the container for nucleic acid amplification reaction.

15. The cartridge for nucleic acid amplification reaction according to any one of claims 9 to 14,
wherein a tip end of the tube on the side on which the fifth plug is disposed is brought into contact with the oil in the container for nucleic acid amplification reaction.

16. The cartridge for nucleic acid amplification reaction according to any one of claims 9 to 15,
wherein the eluent contains a primer for nucleic acid amplification reaction.

17. The cartridge for nucleic acid amplification reaction according to any one of the preceding claims 9 to 16,
wherein the nucleic acid amplification reaction reagent contains a reverse transcriptase.

18. The cartridge for nucleic acid amplification reaction according to any one of claims 9 to 17,
wherein the amount of the nucleic acid amplification reaction reagent solution for freeze-drying the nucleic acid amplification reaction reagent is smaller than the amount of the eluent.

19. A cartridge kit for nucleic acid amplification reaction comprising:
the cartridge for nucleic acid amplification reaction according to any one of claims 9 to 18; and
a tank for introducing a nucleic acid-binding solid-phase carrier into the tube.

20. The cartridge kit for nucleic acid amplification reaction according to Claim 19,
wherein the tank contains a solution for extracting nucleic acids and the nucleic acid-binding solid-phase carrier.

21. The cartridge kit for nucleic acid amplification reaction according to Claim 19 or 20,
wherein the tank has an opening portion and a removable cap on the opening portion.

22. The cartridge kit for nucleic acid amplification reaction according to any one of claims 19 to 21,
wherein the opening portion of the tank is configured to be mountable on the opening portion of the tube on the side on which the first plug is disposed.


SEQUENCE LISTING

<110> Seiko Epson Corporation

<120> Chamber, cartridge, and cartridge kit for Nucleic Acid
Amplification Reaction

<130> J017751501

<160> 3

<170> PatentIn version 3.5

<210> 1
<211> 22
<212> DNA
<213> Influenza A virus

<400> 1
gaccaatcct gtcacctctg ac                                              22

<210> 2
<211> 24
<212> DNA

<213> Influenza A virus

<400> 2

agggcatttt ggacaaagcg tcta 24

<210> 3

<211> 24

<212> DNA

<213> Influenza A virus

<400> 3

tgcagtcctc gctcactggg cacg 24

SEQUENCE LISTING

<110> Seiko Epson Corporation

<120> Chamber, cartridge, and cartridge kit for Nucleic Acid Amplification
Reaction

<130> J017751501

<160> 3

<170> PatentIn version 3.5

<210> 1
<211> 22
<212> DNA
<213> Influenza A virus

<400> 1
gaccaatcct gtcacctctg ac                                                22

<210> 2
<211> 24
<212> DNA
<213> Influenza A virus

<400> 2
agggcatttt ggacaaagcg tcta                                              24

<210> 3
<211> 24
<212> DNA
<213> Influenza A virus

<400> 3
tgcagtcctc gctcactggg cacg                                              24